# EUROPEAN PATENT SPECIFICATION

(11) **EP 3 426 795 B1**
(45) Date of publication and mention of the grant of the patent: **27.01.2021**
(21) Application number: 17711290.1
(22) Date of filing: 10.03.2017
(51) Int. Cl.: C12Q 1/48

(54) **PRENYLATION ASSAY**
PRENYLIERUNGSTEST
DOSAGE DE LA PRÉNYLATION

(30) Priority: 10.03.2016 GB 201604146
(43) Date of publication of application: 16.01.2019
(73) Proprietor: NightstaRx Limited, London NW1 2BE (GB)
(72) Inventor: MACLAREN, Robert E., Oxford Oxfordshire OX3 9DU (GB); MOREIRA PATRICIO, Maria Ines, Oxford Oxfordshire OX3 9DU (GB)
(74) Representative: Adams, Harvey Vaughan John
(86) International application number: PCT/GB2017/050659
(87) International publication number: WO 2017/153774

(56) References cited:
- CREMERS F P ET AL: "REP-2, a Rab escort protein encoded by the choroideremia-like gene.", THE JOURNAL OF BIOLOGICAL CHEMISTRY, vol. 269, no. 3, 21 January 1994 (1994-01-21), pages 2111-2117, XP002769899, ISSN: 0021-9258
- VASIREDDY VIDYULLATHA ET AL: "AAV-mediated gene therapy for choroideremia: preclinical studies in personalized models.", PLOS ONE, vol. 8, no. 5, E61396, 7 May 2013 (2013-05-07), pages 1-13, XP002769900, ISSN: 1932-6203, DOI: 10.1371/journal.pone.0061396
- AYMELT ITZEN ET AL: "GTPases involved in vesicular trafficking: Structures and mechanisms", SEMINARS IN CELL AND DEVELOPMENTAL BIOLOGY, vol. 22, no. 1, 2011, pages 48-56, XP028136567, ISSN: 1084-9521, DOI: 10.1016/J.SEMCDB.2010.10.003
- BERANGER FLORENCE ET AL: "Determination of structural requirements for the interaction of Rab6 with RabGDI and Rab geranylgeranyltransferase", JOURNAL OF BIOLOGICAL CHEMISTRY, vol. 269, no. 18, 6 May 1994 (1994-05-06), pages 13637-13643, XP002769903, ISSN: 0021-9258

### SEQUENCE LISTING

<110> NightstaRx Limited
<120> PRENYLATION ASSAY
<130> P108257PCT
<150> GB 1604146.9
   <151> 2016-03-10
<160> 9
<170> PatentIn version 3.5
<210> 1
   <211> 208
   <212> PRT
   <213> Homo sapiens
<400> 1
   Met Ser Thr Gly Gly Asp Phe Gly Asn Pro Leu Arg Lys Phe Lys Leu
   1               5                   10                  15
   Val Phe Leu Gly Glu Gln Ser Val Gly Lys Thr Ser Leu Ile Thr Arg
               20                  25                  30
   Phe Met Tyr Asp Ser Phe Asp Asn Thr Tyr Gln Ala Thr Ile Gly Ile
           35                  40                  45
   Asp Phe Leu Ser Lys Thr Met Tyr Leu Glu Asp Arg Thr Val Arg Leu
       50                  55                  60
   Gln Leu Trp Asp Thr Ala Gly Gln Glu Arg Phe Arg Ser Leu Ile Pro
   65                  70                  75                  80
   Ser Tyr Ile Arg Asp Ser Thr Val Ala Val Val Val Tyr Asp Ile Thr
                   85                  90                  95
   Asn Val Asn Ser Phe Gln Gln Thr Thr Lys Trp Ile Asp Asp Val Arg
               100                 105                 110
   Thr Glu Arg Gly Ser Asp Val Ile Ile Met Leu Val Gly Asn Lys Thr
           115                 120                 125
   Asp Leu Ala Asp Lys Arg Gln Val Ser Ile Glu Glu Gly Glu Arg Lys
       130                 135                 140
   Ala Lys Glu Leu Asn Val Met Phe Ile Glu Thr Ser Ala Lys Ala Gly
   145                 150                 155                 160
   Tyr Asn Val Lys Gln Leu Phe Arg Arg Val Ala Ala Ala Leu Pro Gly
                   165                 170                 175
   Met Glu Ser Thr Gln Asp Arg Ser Arg Glu Asp Met Ile Asp Ile Lys
               180                 185                 190
   Leu Glu Lys Pro Gln Glu Gln Pro Val Ser Glu Gly Gly Cys Ser Cys
           195                 200                 205
<210> 2
   <211> 627
   <212> DNA
   <213> Homo sapiens
<400> 2
   atgtccacgg gcggagactt cgggaatccg ctgaggaaat tcaagctggt gttcctgggg        60
   gagcaaagcg ttggaaagac atctttgatc accagattca tgtatgacag ttttgacaac       120
   acctatcagg caacaattgg cattgacttt ttatcaaaaa ctatgtactt ggaggatcga       180
   acagtacgat tgcaattatg ggacacagca ggtcaagagc ggttcaggag cttgattcct       240
   agctacattc gtgactccac tgtggcagtt gttgtttatg atatcacaaa tgttaactca       300
   ttccagcaaa ctacaaagtg gattgatgat gtcagaacag aaagaggaag tgatgttatc       360
   atcatgctag taggaaataa aacagatctt gctgacaaga ggcaagtgtc aattgaggag       420
   ggagagagga aagccaaaga gctgaatgtt atgtttattg aaactagtgc aaaagctgga       480
   tacaatgtaa agcagctctt tcgacgtgta gcagcagctt tgccgggaat ggaaagcaca       540
   caggacagaa gcagagaaga tatgattgac ataaaactgg aaaagcctca ggagcaacca       600
   gtcagtgaag gaggctgttc ctgctaa                                           627
<210> 3
   <211> 567
   <212> PRT
   <213> Homo sapiens
<400> 3
   Met His Gly Arg Leu Lys Val Lys Thr Ser Glu Glu Gln Ala Glu Ala
   1               5                   10                  15
   Lys Arg Leu Glu Arg Glu Gln Lys Leu Lys Leu Tyr Gln Ser Ala Thr
               20                  25                  30
   Gln Ala Val Phe Gln Lys Arg Gln Ala Gly Glu Leu Asp Glu Ser Val
           35                  40                  45
   Leu Glu Leu Thr Ser Gln Ile Leu Gly Ala Asn Pro Asp Phe Ala Thr
       50                  55                  60
   Leu Trp Asn Cys Arg Arg Glu Val Leu Gln Gln Leu Glu Thr Gln Lys
   65 70 75 80
   Ser Pro Glu Glu Leu Ala Ala Leu Val Lys Ala Glu Leu Gly Phe Leu
   85 90 95
   Glu Ser Cys Leu Arg Val Asn Pro Lys Ser Tyr Gly Thr Trp His His
   100 105 110
   Arg Cys Trp Leu Leu Gly Arg Leu Pro Glu Pro Asn Trp Thr Arg Glu
   115 120 125
   Leu Glu Leu Cys Ala Arg Phe Leu Glu Val Asp Glu Arg Asn Phe His
   130 135 140
   Cys Trp Asp Tyr Arg Arg Phe Val Ala Thr Gln Ala Ala Val Pro Pro
   145 150 155 160
   Ala Glu Glu Leu Ala Phe Thr Asp Ser Leu Ile Thr Arg Asn Phe Ser
   165 170 175
   Asn Tyr Ser Ser Trp His Tyr Arg Ser Cys Leu Leu Pro Gln Leu His
   180 185 190
   Pro Gln Pro Asp Ser Gly Pro Gln Gly Arg Leu Pro Glu Asp Val Leu
   195 200 205
   Leu Lys Glu Leu Glu Leu Val Gln Asn Ala Phe Phe Thr Asp Pro Asn
   210 215 220
   Asp Gln Ser Ala Trp Phe Tyr His Arg Trp Leu Leu Gly Arg Ala Asp
   225 230 235 240
   Pro Gln Asp Ala Leu Arg Cys Leu His Val Ser Arg Asp Glu Ala Cys
   245 250 255
   Leu Thr Val Ser Phe Ser Arg Pro Leu Leu Val Gly Ser Arg Met Glu
   260 265 270
   Ile Leu Leu Leu Met Val Asp Asp Ser Pro Leu Ile Val Glu Trp Arg
   275 280 285
   Thr Pro Asp Gly Arg Asn Arg Pro Ser His Val Trp Leu Cys Asp Leu
   290 295 300
   Pro Ala Ala Ser Leu Asn Asp Gln Leu Pro Gln His Thr Phe Arg Val
   305 310 315 320
   Ile Trp Thr Ala Gly Asp Val Gln Lys Glu Cys Val Leu Leu Lys Gly
                   325                 330                 335
   Arg Gln Glu Gly Trp Cys Arg Asp Ser Thr Thr Asp Glu Gln Leu Phe
               340                 345                 350
   Arg Cys Glu Leu Ser Val Glu Lys Ser Thr Val Leu Gln Ser Glu Leu
           355                 360                 365
   Glu Ser Cys Lys Glu Leu Gln Glu Leu Glu Pro Glu Asn Lys Trp Cys
       370                 375                 380
   Leu Leu Thr Ile Ile Leu Leu Met Arg Ala Leu Asp Pro Leu Leu Tyr
   385                 390                 395                 400
   Glu Lys Glu Thr Leu Gln Tyr Phe Gln Thr Leu Lys Ala Val Asp Pro
                   405                 410                 415
   Met Arg Ala Thr Tyr Leu Asp Asp Leu Arg Ser Lys Phe Leu Leu Glu
               420                 425                 430
   Asn Ser Val Leu Lys Met Glu Tyr Ala Glu Val Arg Val Leu His Leu
           435                 440                 445
   Ala His Lys Asp Leu Thr Val Leu Cys His Leu Glu Gln Leu Leu Leu
       450                 455                 460
   Val Thr His Leu Asp Leu Ser His Asn Arg Leu Arg Thr Leu Pro Pro
   465                 470                 475                 480
   Ala Leu Ala Ala Leu Arg Cys Leu Glu Val Leu Gln Ala Ser Asp Asn
                   485                 490                 495
   Ala Ile Glu Ser Leu Asp Gly Val Thr Asn Leu Pro Arg Leu Gln Glu
               500                 505                 510
   Leu Leu Leu Cys Asn Asn Arg Leu Gln Gln Pro Ala Val Leu Gln Pro
           515                 520                 525
   Leu Ala Ser Cys Pro Arg Leu Val Leu Leu Asn Leu Gln Gly Asn Pro
       530                 535                 540
   Leu Cys Gln Ala Val Gly Ile Leu Glu Gln Leu Ala Glu Leu Leu Pro
   545                 550                 555                 560
   Ser Val Ser Ser Val Leu Thr
                   565
<210> 4
   <211> 331
   <212> PRT
   <213> Homo sapiens
<400> 4
   Met Gly Thr Pro Gln Lys Asp Val Ile Ile Lys Ser Asp Ala Pro Asp
   1               5                   10                  15
   Thr Leu Leu Leu Glu Lys His Ala Asp Tyr Ile Ala Ser Tyr Gly Ser
               20                  25                  30
   Lys Lys Asp Asp Tyr Glu Tyr Cys Met Ser Glu Tyr Leu Arg Met Ser
           35                  40                  45
   Gly Ile Tyr Trp Gly Leu Thr Val Met Asp Leu Met Gly Gln Leu His
       50                  55                  60
   Arg Met Asn Arg Glu Glu Ile Leu Ala Phe Ile Lys Ser Cys Gln His
   65                  70                  75                  80
   Glu Cys Gly Gly Ile Ser Ala Ser Ile Gly His Asp Pro His Leu Leu
                   85                  90                  95
   Tyr Thr Leu Ser Ala Val Gln Ile Leu Thr Leu Tyr Asp Ser Ile Asn
               100                 105                 110
   Val Ile Asp Val Asn Lys Val Val Glu Tyr Val Lys Gly Leu Gln Lys
           115                 120                 125
   Glu Asp Gly Ser Phe Ala Gly Asp Ile Trp Gly Glu Ile Asp Thr Arg
       130                 135                 140
   Phe Ser Phe Cys Ala Val Ala Thr Leu Ala Leu Leu Gly Lys Leu Asp
   145                 150                 155                 160
   Ala Ile Asn Val Glu Lys Ala Ile Glu Phe Val Leu Ser Cys Met Asn
                   165                 170                 175
   Phe Asp Gly Gly Phe Gly Cys Arg Pro Gly Ser Glu Ser His Ala Gly
               180                 185                 190
   Gln Ile Tyr Cys Cys Thr Gly Phe Leu Ala Ile Thr Ser Gln Leu His
           195                 200                 205
   Gln Val Asn Ser Asp Leu Leu Gly Trp Trp Leu Cys Glu Arg Gln Leu
       210                 215                 220
   Pro Ser Gly Gly Leu Asn Gly Arg Pro Glu Lys Leu Pro Asp Val Cys
   225                 230                 235                 240
   Tyr Ser Trp Trp Val Leu Ala Ser Leu Lys Ile Ile Gly Arg Leu His
                   245                 250                 255
   Trp Ile Asp Arg Glu Lys Leu Arg Asn Phe Ile Leu Ala Cys Gln Asp
               260                 265                 270
   Glu Glu Thr Gly Gly Phe Ala Asp Arg Pro Gly Asp Met Val Asp Pro
           275                 280                 285
   Phe His Thr Leu Phe Gly Ile Ala Gly Leu Ser Leu Leu Gly Glu Glu
       290                 295                 300
   Gln Ile Lys Pro Val Asn Pro Val Phe Cys Met Pro Glu Glu Val Leu
   305                 310                 315                 320
   Gln Arg Val Asn Val Gln Pro Glu Leu Val Ser
                   325                 330
<210> 5
   <211> 653
   <212> PRT
   <213> Homo sapiens
<400> 5
<210> 6
   <211> 1962
   <212> DNA
   <213> Homo sapiens
<400> 6
   atggcggata ctctcccttc ggagtttgat gtgatcgtaa tagggacggg tttgcctgaa       60
   tccatcattg cagctgcatg ttcaagaagt ggccggagag ttctgcatgt tgattcaaga      120
   agctactatg gaggaaactg ggccagtttt agcttttcag gactattgtc ctggctaaag      180
   gaataccagg aaaacagtga cattgtaagt gacagtccag tgtggcaaga ccagatcctt      240
   gaaaatgaag aagccattgc tcttagcagg aaggacaaaa ctattcaaca tgtggaagta      300
   ttttgttatg ccagtcagga tttgcatgaa gatgtcgaag aagctggtgc actgcagaaa      360
   aatcatgctc ttgtgacatc tgcaaactcc acagaagctg cagattctgc cttcctgcct      420
   acggaggatg agtcattaag cactatgagc tgtgaaatgc tcacagaaca aactccaagc      480
   agcgatccag agaatgcgct agaagtaaat ggtgctgaag tgacagggga aaaagaaaac      540
   cattgtgatg ataaaacttg tgtgccatca acttcagcag aagacatgag tgaaaatgtg      600
   cctatagcag aagataccac agagcaacca aagaaaaaca gaattactta ctcacaaatt      660
   attaaagaag gcaggagatt taatattgat ttagtatcaa agctgctgta ttctcgagga      720
   ttactaattg atcttctaat caaatctaat gttagtcgat atgcagagtt taaaaatatt      780
   accaggattc ttgcatttcg agaaggacga gtggaacagg ttccgtgttc cagagcagat      840
   gtctttaata gcaaacaact tactatggta gaaaagcgaa tgctaatgaa atttcttaca      900
   ttttgtatgg aatatgagaa atatcctgat gaatataaag gatatgaaga gatcacattt      960
   tatgaatatt taaagactca aaaattaacc cccaacctcc aatatattgt catgcattca     1020
   attgcaatga catcagagac agccagcagc accatagatg gtctcaaagc taccaaaaac     1080
   tttcttcact gtcttgggcg gtatggcaac actccatttt tgtttccttt atatggccaa     1140
   ggagaactcc cccagtgttt ctgcaggatg tgtgctgtgt ttggtggaat ttattgtctt     1200
   cgccattcag tacagtgcct tgtagtggac aaagaatcca gaaaatgtaa agcaattata     1260
   gatcagtttg gtcagagaat aatctctgag catttcctcg tggaggacag ttactttcct     1320
   gagaacatgt gctcacgtgt gcaatacagg cagatctcca gggcagtgct gattacagat     1380
   agatctgtcc taaaaacaga ttcagatcaa cagatttcca ttttgacagt gccagcagag     1440
   gaaccaggaa cttttgctgt tcgggtcatt gagttatgtt cttcaacgat gacatgcatg     1500
   aaaggcacct atttggttca tttgacttgc acatcttcta aaacagcaag agaagattta     1560
   gaatcagttg tgcagaaatt gtttgttcca tatactgaaa tggagataga aaatgaacaa     1620
   gtagaaaagc caagaattct gtgggctctt tacttcaata tgagagattc gtcagacatc     1680
   agcaggagct gttataatga tttaccatcc aacgtttatg tctgctctgg cccagattgt     1740
   ggtttaggaa atgataatgc agtcaaacag gctgaaacac ttttccagga aatctgcccc     1800
   aatgaagatt tctgtccccc tccaccaaat cctgaagaca ttatccttga tggagacagt     1860
   ttacagccag aggcttcaga atccagtgcc ataccagagg ctaactcgga gactttcaag     1920
   gaaagcacaa accttggaaa cctagaggag tcctctgaat aa                        1962
<210> 7
   <211> 1992
   <212> DNA
   <213> Homo sapiens
<400> 7
   gatatcgaat tcctgcagcc cggcggcacc atggcggata ctctcccttc ggagtttgat 60
   gtgatcgtaa tagggacggg tttgcctgaa tccatcattg cagctgcatg ttcaagaagt 120
   ggccggagag ttctgcatgt tgattcaaga agctactatg gaggaaactg ggccagtttt 180
   agcttttcag gactattgtc ctggctaaag gaataccagg aaaacagtga cattgtaagt 240
   gacagtccag tgtggcaaga ccagatcctt gaaaatgaag aagccattgc tcttagcagg 300
   aaggacaaaa ctattcaaca tgtggaagta ttttgttatg ccagtcagga tttgcatgaa 360
   gatgtcgaag aagctggtgc actgcagaaa aatcatgctc ttgtgacatc tgcaaactcc 420
   acagaagctg cagattctgc cttcctgcct acggaggatg agtcattaag cactatgagc 480
   tgtgaaatgc tcacagaaca aactccaagc agcgatccag agaatgcgct agaagtaaat 540
   ggtgctgaag tgacagggga aaaagaaaac cattgtgatg ataaaacttg tgtgccatca 600
   acttcagcag aagacatgag tgaaaatgtg cctatagcag aagataccac agagcaacca 660
   aagaaaaaca gaattactta ctcacaaatt attaaagaag gcaggagatt taatattgat 720
   ttagtatcaa agctgctgta ttctcgagga ttactaattg atcttctaat caaatctaat 780
   gttagtcgat atgcagagtt taaaaatatt accaggattc ttgcatttcg agaaggacga 840
   gtggaacagg ttccgtgttc cagagcagat gtctttaata gcaaacaact tactatggta 900
   gaaaagcgaa tgctaatgaa atttcttaca ttttgtatgg aatatgagaa atatcctgat 960
   gaatataaag gatatgaaga gatcacattt tatgaatatt taaagactca aaaattaacc 1020
   cccaacctcc aatatattgt catgcattca attgcaatga catcagagac agccagcagc 1080
   accatagatg gtctcaaagc taccaaaaac tttcttcact gtcttgggcg gtatggcaac 1140
   actccatttt tgtttccttt atatggccaa ggagaactcc cccagtgttt ctgcaggatg 1200
   tgtgctgtgt ttggtggaat ttattgtctt cgccattcag tacagtgcct tgtagtggac 1260
   aaagaatcca gaaaatgtaa agcaattata gatcagtttg gtcagagaat aatctctgag 1320
   catttcctcg tggaggacag ttactttcct gagaacatgt gctcacgtgt gcaatacagg 1380
   cagatctcca gggcagtgct gattacagat agatctgtcc taaaaacaga ttcagatcaa 1440
   cagatttcca ttttgacagt gccagcagag gaaccaggaa cttttgctgt tcgggtcatt 1500
   gagttatgtt cttcaacgat gacatgcatg aaaggcacct atttggttca tttgacttgc 1560
   acatcttcta aaacagcaag agaagattta gaatcagttg tgcagaaatt gtttgttcca 1620
   tatactgaaa tggagataga aaatgaacaa gtagaaaagc caagaattct gtgggctctt 1680
   tacttcaata tgagagattc gtcagacatc agcaggagct gttataatga tttaccatcc 1740
   aacgtttatg tctgctctgg cccagattgt ggtttaggaa atgataatgc agtcaaacag 1800
   gctgaaacac ttttccagga aatctgcccc aatgaagatt tctgtccccc tccaccaaat 1860
   cctgaagaca ttatccttga tggagacagt ttacagccag aggcttcaga atccagtgcc 1920
   ataccagagg ctaactcgga gactttcaag gaaagcacaa accttggaaa cctagaggag 1980
   tcctctgaat aa 1992
<210> 8
   <211> 567
   <212> PRT
   <213> Rattus norvegicus
<400> 8
   Met His Gly Arg Leu Lys Val Lys Thr Ser Glu Glu Gln Ala Glu Ala
   1               5                   10                  15
   Lys Arg Leu Glu Arg Glu Gln Lys Leu Lys Leu Tyr Gln Ser Ala Thr
               20                  25                  30
   Gln Ala Val Phe Gln Lys Arg Gln Ala Gly Glu Leu Asp Glu Ser Val
           35                  40                  45
   Leu Glu Leu Thr Ser Gln Ile Leu Gly Ala Asn Pro Asp Phe Ala Thr
       50                  55                  60
   Leu Trp Asn Cys Arg Arg Glu Val Leu Gln His Leu Glu Thr Glu Lys
   65                  70                  75                  80
   Ser Pro Glu Glu Ser Ala Ala Leu Val Lys Ala Glu Leu Gly Phe Leu
                   85                  90                  95
   Glu Ser Cys Leu Arg Val Asn Pro Lys Ser Tyr Gly Thr Trp His His
               100                 105                 110
   Arg Cys Trp Leu Leu Ser Arg Leu Pro Glu Pro Asn Trp Ala Arg Glu
           115                 120                 125
   Leu Glu Leu Cys Ala Arg Phe Leu Glu Ala Asp Glu Arg Asn Phe His
       130                 135                 140
   Cys Trp Asp Tyr Arg Arg Phe Val Ala Ala Gln Ala Ala Val Ala Pro
   145                 150                 155                 160
   Ala Glu Glu Leu Ala Phe Thr Asp Ser Leu Ile Thr Arg Asn Phe Ser
                   165                 170                 175
   Asn Tyr Ser Ser Trp His Tyr Arg Ser Cys Leu Leu Pro Gln Leu His
               180                 185                 190
   Pro Gln Pro Asp Ser Gly Pro Gln Gly Arg Leu Pro Glu Asn Val Leu
           195                 200                 205
   Leu Lys Glu Leu Glu Leu Val Gln Asn Ala Phe Phe Thr Asp Pro Asn
       210                 215                 220
   Asp Gln Ser Ala Trp Phe Tyr His Arg Trp Leu Leu Gly Arg Ala Glu
   225                 230                 235                 240
   Pro His Asp Val Leu Cys Cys Val His Val Ser Arg Glu Glu Ala Cys
                   245                 250                 255
   Leu Ser Val Cys Phe Ser Arg Pro Leu Thr Val Gly Ser Arg Met Gly
               260                 265                 270
   Thr Leu Leu Leu Met Val Asp Glu Ala Pro Leu Ser Val Glu Trp Arg
           275                 280                 285
   Thr Pro Asp Gly Arg Asn Arg Pro Ser His Val Trp Leu Cys Asp Leu
       290                 295                 300
   Pro Ala Ala Ser Leu Asn Asp Gln Leu Pro Gln His Thr Phe Arg Val
   305                 310                 315                 320
   Ile Trp Thr Gly Ser Asp Ser Gln Lys Glu Cys Val Leu Leu Lys Asp
                   325                 330                 335
   Arg Pro Glu Cys Trp Cys Arg Asp Ser Ala Thr Asp Glu Gln Leu Phe
   340 345 350
   Arg Cys Glu Leu Ser Val Glu Lys Ser Thr Val Leu Gln Ser Glu Leu
   355 360 365
   Glu Ser Cys Lys Glu Leu Gln Glu Leu Glu Pro Glu Asn Lys Trp Cys
   370 375 380
   Leu Leu Thr Ile Ile Leu Leu Met Arg Ala Leu Asp Pro Leu Leu Tyr
   385 390 395 400
   Glu Lys Glu Thr Leu Gln Tyr Phe Ser Thr Leu Lys Ala Val Asp Pro
   405 410 415
   Met Arg Ala Ala Tyr Leu Asp Asp Leu Arg Ser Lys Phe Leu Leu Glu
   420 425 430
   Asn Ser Val Leu Lys Met Glu Tyr Ala Asp Val Arg Val Leu His Leu
   435 440 445
   Ala His Lys Asp Leu Thr Val Leu Cys His Leu Glu Gln Leu Leu Leu
   450 455 460
   Val Thr His Leu Asp Leu Ser His Asn Arg Leu Arg Ala Leu Pro Pro
   465 470 475 480
   Ala Leu Ala Ala Leu Arg Cys Leu Glu Val Leu Gln Ala Ser Asp Asn
   485 490 495
   Ala Leu Glu Asn Val Asp Gly Val Ala Asn Leu Pro Arg Leu Gln Glu
   500 505 510
   Leu Leu Leu Cys Asn Asn Arg Leu Gln Gln Ser Ala Ala Ile Gln Pro
   515 520 525
   Leu Val Ser Cys Pro Arg Leu Val Leu Leu Asn Leu Gln Gly Asn Ser
   530 535 540
   Leu Cys Gln Glu Glu Gly Ile Gln Glu Arg Leu Ala Glu Met Leu Pro
   545 550 555 560
   Ser Val Ser Ser Ile Leu Thr
   565
<210> 9
   <211> 331
   <212> PRT
   <213> Rattus norvegicus
<400> 9
   Met Gly Thr Gln Gln Lys Asp Val Thr Ile Lys Ser Asp Ala Pro Asp
   1               5                   10                  15
   Thr Leu Leu Leu Glu Lys His Ala Asp Tyr Ile Ala Ser Tyr Gly Ser
               20                  25                  30
   Lys Lys Asp Asp Tyr Glu Tyr Cys Met Ser Glu Tyr Leu Arg Met Ser
           35                  40                  45
   Gly Val Tyr Trp Gly Leu Thr Val Met Asp Leu Met Gly Gln Leu His
       50                  55                  60
   Arg Met Asn Lys Glu Glu Ile Leu Val Phe Ile Lys Ser Cys Gln His
   65                  70                  75                  80
   Glu Cys Gly Gly Val Ser Ala Ser Ile Gly His Asp Pro His Leu Leu
                   85                  90                  95
   Tyr Thr Leu Ser Ala Val Gln Ile Leu Thr Leu Tyr Asp Ser Ile His
               100                 105                 110
   Val Ile Asn Val Asp Lys Val Val Ala Tyr Val Gln Ser Leu Gln Lys
           115                 120                 125
   Glu Asp Gly Ser Phe Ala Gly Asp Ile Trp Gly Glu Ile Asp Thr Arg
       130                 135                 140
   Phe Ser Phe Cys Ala Val Ala Thr Leu Ala Leu Leu Gly Lys Leu Asp
   145                 150                 155                 160
   Ala Ile Asn Val Glu Lys Ala Ile Glu Phe Val Leu Ser Cys Met Asn
                   165                 170                 175
   Phe Asp Gly Gly Phe Gly Cys Arg Pro Gly Ser Glu Ser His Ala Gly
               180                 185                 190
   Gln Ile Tyr Cys Cys Thr Gly Phe Leu Ala Ile Thr Ser Gln Leu His
           195                 200                 205
   Gln Val Asn Ser Asp Leu Leu Gly Trp Trp Leu Cys Glu Arg Gln Leu
       210                 215                 220
   Pro Ser Gly Gly Leu Asn Gly Arg Pro Glu Lys Leu Pro Asp Val Cys
   225                 230                 235                 240
   Tyr Ser Trp Trp Val Leu Ala Ser Leu Lys Ile Ile Gly Arg Leu His
                   245                 250                 255
   Trp Ile Asp Arg Glu Lys Leu Arg Ser Phe Ile Leu Ala Cys Gln Asp
               260                 265                 270
   Glu Glu Thr Gly Gly Phe Ala Asp Arg Pro Gly Asp Met Val Asp Pro
           275                 280                 285
   Phe His Thr Leu Phe Gly Ile Ala Gly Leu Ser Leu Leu Gly Glu Glu
       290                 295                 300
   Gln Ile Lys Pro Val Ser Pro Val Phe Cys Met Pro Glu Glu Val Leu
   305                 310                 315                 320
   Gln Arg Val Asn Val Gln Pro Glu Leu Val Ser
                   325                 330

## Description

### FIELD

The present disclosure relates to an assay for use in determining the activity of Rab escort protein 1 (REP1). More specifically, the disclosure relates to the use of Rab6a in an assay as a substrate for prenylation, in particular wherein the REP1 has been delivered to a cell using a gene therapy vector.

### BACKGROUND

Choroideremia is a rare disease which leads to degeneration of the choroid, retinal pigment epithelium and photoreceptors of the eye. Afflicted males typically exhibit nightblindness during teenage years, progressive loss of peripheral vision during the 20's and 30's and complete blindness in the 40's. Female carriers have mild symptoms, most notably nightblindness, but may occasionally have a more severe phenotype.

Choroideremia is caused by mutations in the *CHM* gene, which encodes for Rab escort protein 1 (REP1). Rab escort protein 2 (REP2), which is 75% homologous to REP1, compensates for any REP1 deficiency in most cells of the body. However, REP2 is unable to compensate for REP1 deficiency in the eye. This leads to insufficient Rab escort protein activity to maintain normal prenylation of target Rab GTPases and gives rise to cellular dysfunction and ultimately cell death.

Choroideremia may be successfully treated by providing functional copies of the REP1 transgene to the affected cells of the eye (MacLaren, R.E. et al. (2014) Lancet 383: 1129-37). Specifically, it has been shown that adeno-associated virus (AAV) gene therapy vectors may be used to deliver a nucleotide sequence encoding functional REP1 to the eye to treat the disease.

As gene therapy of choroideremia is becoming a clinical reality, there is a need for reliable and sensitive assays to determine the activity of exogenously delivered REP1, in particular to test new gene therapy vectors and as a quality control screen for clinical vector stocks.

Cremers, F.P. et al. (1994) J Biol Chem 269(3():2111-2117 reported that REP1 and REP2 were approximately equal in facilitating the attachment of GG groups to several Rab proteins, including Rab1A, Rab5A, and Rab6. Existing methods for assaying REP1 use Rab27a as a prenylation substrate (Tolmachova, T. et al. (2012) J. Gene Med. 14: 158-168; Tolmachova, T. et al. (2013) J. Mol. Med. 91: 825-837; Vasireddy, V. et al. (2013) PLoS ONE 8: e61396; and Black, A. et al. (2014) J. Gene Med. 16: 122-130). This has likely followed from numerous implications of Rab27a in the pathogenesis of choroideremia. For example, it has been shown that Rab27a is present unprenylated in choroideremia cells while other Rabs are properly prenylated (Seabra, M.C. et al. (1995) J. Biol. Chem. 270: 24420-24427). Furthermore, Rab27a is expressed at high levels in the retinal pigment epithelium and choriocapillaries, the two sites of earliest degeneration in choroideremia.

However, assays relying on the prenylation of Rab27a give rise to very weak signals. As a result, the sensitivity of these assays is low and they may not be suitable for reliable screening of clinical gene therapy vectors. Accordingly, a significant need exists for more reliable and sensitive assays which can be used to determine REP1 activity and test gene therapy vectors.

### SUMMARY

The present invention provides a method for determining the activity of a Rab escort protein 1 (REP1)-encoding gene therapy vector for the use in the treatment of choroideremia, or a method for quality control analysis of a Rab escort protein 1 (REP1)-encoding gene therapy vector for the use in the treatment of choroideremia; the method comprising the steps of: (a) contacting a sample comprising REP1 with Rab6a, Rab geranylgeranyltransferase (Rab GGTase) and a lipid donor substrate, wherein the sample comprising REP1 is from a cell genetically engineered to express the REP1 using the REP1-encoding gene therapy vector; and (b) detecting the lipidated Rab6a product.

The present invention also provides use of Rab6a for determining in vitro the activity of a Rab escort protein 1 (REP1)-encoding gene therapy vector for use in the treatment of choroideremia, or use of Rab6a for in vitro quality control analysis of a Rab escort protein 1 (REP1)-encoding gene therapy vector for the use in the treatment of choroideremia; wherein the Rab6a is contacted with REP1 from a cell genetically engineered to express the REP1 using the REP1-encoding gene therapy vector.

Further aspects and embodiments of the invention are set out in the claims.

The present inventors have surprisingly found that, despite a prevailing understanding that Rab27a provides the most suitable prenylation assay substrate, use of Rab6a as a substrate in a prenylation reaction provides a more sensitive method for determining the activity of Rab escort protein 1 (REP1). Not only does Rab6a provide for increased sensitivity in an assay detecting prenylation, it also provides for beneficial signal-to-noise ratios, better dynamic range of signal and better consistency.

Moreover, the inventors have demonstrated that the increased sensitivity of a Rab6a-based assay may be harnessed to accurately and reliably determine the activity of REP1-encoding vectors, in particular AAV gene therapy vectors, such as those suitable for use in the clinic.

Accordingly, in one aspect, the disclosure provides a method for determining the activity of Rab escort protein 1 (REP1) comprising the steps:
(a) providing a sample comprising REP1;
(b) contacting the sample of step (a) with Rab6a, Rab geranylgeranyltransferase (Rab GGTase) and a lipid donor substrate; and
(c) detecting the lipidated Rab6a product.

For example, the method of the disclosure may be for testing gene therapy vectors suitable for the delivery of REP1 to a target cell or for quality control analysis of vector stocks (e.g. medicament stocks).

Validation of gene therapy vectors is mandatory for the safe and efficacious implementation of gene therapy in the clinic. For analysis and quality control steps, comparison with control experiments or reference levels may provide a measure of the activity of the gene therapy vector or REP1 relative to a known or accepted standard (e.g. better or worse than a known or accepted standard). This may be used to validate whether a gene therapy vector stock meets specific targets and regulations.

In one instance, comparison is made to a sample of REP1 or REP1-encoding AAV vector that is defined as a primary reference standard. The method of the disclosure may be, for example, carried out in parallel on a test sample and the primary reference standard sample. Potency and/or behaviour of the test sample may be, for example, defined relative to the primary reference standard.

Put another way, the method of the disclosure may, for example, be used for quality control analysis and validation of a gene therapy vector as efficacious (e.g. for the treatment of choroideremia), preferably an AAV vector particle comprising a REP1-encoding nucleotide sequence, preferably wherein an output activity or efficacy of the vector determined by the method of the disclosure above a threshold activity or within a specified target range (e.g. by comparison to a control experiment or reference level) indicates the vector is suitable for gene therapy purposes.

Accordingly, in another aspect, the method of the disclosure is for quality control analysis of a Rab escort protein 1 (REP1)-encoding gene therapy vector (preferably an AAV vector).

In another aspect, the disclosure provides a method for quality control analysis of a Rab escort protein 1 (REP1)-encoding gene therapy vector (preferably an AAV vector) comprising the steps:
(a) transducing a cell with the vector, culturing the cell under conditions suitable for the expression of the REP1 and lysing the cells to provide a sample comprising REP1;
(b) contacting the sample of step (a) with Rab6a, Rab geranylgeranyltransferase (Rab GGTase) and a lipid donor substrate; and
(c) detecting the lipidated Rab6a product.

Accordingly, the method may comprise carrying out a plurality of experiments comprising steps (a) to (c) in which parameters relating to the sample comprising REP1 are varied, while other parameters (e.g. parameters relating to the Rab6a, Rab GGTase and lipid donor substrate) are kept constant. Such parameters may include, for example, the amino acid sequence of the relevant protein (e.g. REP1), the REP1-encoding nucleotide sequence comprised in a vector used to express the REP1 in a cell, the type of vector used to deliver a REP1-encoding nucleotide sequence to a cell (e.g. the type of viral vector, such as the type of adeno-associated viral (AAV) vector), the concentration of REP1 and/or the multiplicity-of-infection (MOI) of a vector used to deliver a REP1-encoding nucleotide sequence to a cell. In a preferred instance, the method comprises carrying out a plurality of experiments comprising steps (a) to (c) at different MOIs of a vector used to deliver a REP1-encoding nucleotide sequence to a cell (e.g. to generate a dose-response curve).

In one instance, the detection of the lipidated Rab6a product comprises quantifying the amount of the lipidated Rab6a product. In a preferred instance, the detection of the lipidated Rab6a product comprises quantifying the amount of the lipidated Rab6a product relative to a control or reference level. The quantification may be, for example, made relative to a sample of REP1 or REP1-encoding AAV vector that is defined as a primary reference standard. The method of the disclosure may be, for example, carried out in parallel on a test sample and the primary reference standard sample. Potency and/or behaviour of the test sample may be, for example, defined relative to the primary reference standard.

In one instance, the method comprises a further step of comparing the amount of lipidated Rab6a product (e.g. prenylated, such as geranylgeranylated or biotin-geranylated, Rab6a) with an amount determined from a control experiment, such as an experiment using a known or standard sample of REP1.

In another instance, the method comprises a further step of comparing the amount of lipidated Rab6a product (e.g. prenylated, such as geranylgeranylated or biotin-geranylated, Rab6a) with a reference level.

In one instance, the sample comprising REP1 is from a cell genetically engineered to express the REP1. Preferably, the sample comprising REP1 is a lysate of a cell genetically engineered to express the REP1. Preferably, a cell is transfected or transduced with a vector comprising a REP1-encoding nucleotide sequence to provide the cell genetically engineered to express the REP1. Preferably, the vector is a viral vector.

In one instance, the REP1 is expressed using a viral vector comprising a REP1-encoding nucleotide sequence.

In one instance, the viral vector is an adeno-associated viral (AAV) vector. Preferably the viral vector is in the form of a viral vector particle.

The AAV vector may be of any serotype (e.g. comprise any AAV serotype genome and/or capsid protein). Preferably, the vector is capable of infecting or transducing cells of the eye.

In one instance, the AAV vector comprises an AAV serotype 1, 2, 3, 4, 5, 6, 7, 8, 9, 10, or 11 genome. In another instance, the AAV vector comprises an AAV serotype 2, 4, 5 or 8 genome. Preferably, the AAV vector comprises an AAV serotype 2 genome.

In one instance, the AAV vector particle comprises an AAV serotype 1, 2, 3, 4, 5, 6, 7, 8, 9, 10, or 11 capsid protein. In another instance, the AAV vector particle comprises an AAV serotype 2, 4, 5 or 8 capsid protein. The AAV serotype 8 capsid protein may, for example, be a AAV8/Y733F mutant capsid protein. Preferably, the AAV vector particle comprises an AAV serotype 2 capsid protein.

In one instance, the AAV vector particle comprises an AAV2 genome and AAV2 capsid proteins (AAV2/2); an AAV2 genome and AAV5 capsid proteins (AAV2/5); or an AAV2 genome and AAV8 capsid proteins (AAV2/8). Preferably, the AAV vector particle comprises an AAV2 genome and AAV2 capsid proteins (AAV2/2).

The AAV vector particle may be a chimeric, shuffled or capsid-modified derivative of one or more naturally occurring AAVs. In particular, the AAV vector particle may comprise capsid protein sequences from different serotypes, clades, clones or isolates of AAV within the same vector (i.e. a pseudotyped vector). Thus, in one instance the AAV vector is in the form of a pseudotyped AAV vector particle.

In one instance, the REP1 is human REP1.

In one instance, the REP1 comprises an amino acid sequence that has at least 70%, 80%, 85%, 90%, 91%, 92%, 93%, 94%, 95%, 96%, 97%, 98%, 99% or 100% identity to SEQ ID NO: 5, preferably wherein the amino acid sequence substantially retains the natural function of the protein represented by SEQ ID NO: 5.

In one instance, the REP1-encoding nucleotide sequence comprises a nucleotide sequence that has at least 70%, 80%, 85%, 90%, 91%, 92%, 93%, 94%, 95%, 96%, 97%, 98%, 99% or 100% identity to SEQ ID NO: 6 or 7, preferably wherein the protein encoded by the nucleotide sequence substantially retains the natural function of the protein represented by SEQ ID NO: 5.

In one instance, the REP1-encoding nucleotide sequence comprises a nucleotide sequence that encodes an amino acid sequence that has at least 70%, 80%, 85%, 90%, 91%, 92%, 93%, 94%, 95%, 96%, 97%, 98%, 99% or 100% identity to SEQ ID NO: 5, preferably wherein the amino acid sequence substantially retains the natural function of the protein represented by SEQ ID NO: 5.

In one instance, the Rab6a and/or Rab GGTase are substantially pure.

In one instance, the Rab6a comprises an amino acid sequence that has at least 70%, 80%, 85%, 90%, 91%, 92%, 93%, 94%, 95%, 96%, 97%, 98%, 99% or 100% identity to SEQ ID NO: 1, preferably wherein the amino acid sequence substantially retains the natural function of the protein represented by SEQ ID NO: 1.

In one instance, the Rab GGTase comprises an amino acid sequence that has at least 70%, 80%, 85%, 90%, 91%, 92%, 93%, 94%, 95%, 96%, 97%, 98%, 99% or 100% identity to SEQ ID NO: 3 or 8, preferably SEQ ID NO: 8, preferably wherein the amino acid sequence substantially retains the natural function of the protein represented by SEQ ID NO: 8; and/or an amino acid sequence that has at least 70%, 80%, 85%, 90%, 91%, 92%, 93%, 94%, 95%, 96%, 97%, 98%, 99% or 100% identity to SEQ ID NO: 4 or 9, preferably SEQ ID NO: 9, preferably wherein the amino acid sequence substantially retains the natural function of the protein represented by SEQ ID NO: 9.

In another instance, the Rab6a:Rab GGTase molar ratio is about 1:0.25-3, 1:0.3-2.9, 1:0.35-2.8, 1:0.4-2.7, 1:0.45-2.6 or 1:0.5-2.5, preferably about 1:0.5-2.5.

In one instance, the Rab6a:Rab GGTase molar ratio is about 1:2-3, 1:2.1-2.9, 1:2.2-2.8, 1:2.3-2.7 or 1:2.4-2.6, preferably about 1:2.4-2.6. In one instance, the Rab6a:Rab GGTase molar ratio is about 1:2, 1:2.1, 1:2.2, 1:2.3, 1:2.4, 1:2.5, 1:2.6, 1:2.7, 1:2.8, 1:2.9 or 1:3, preferably about 1:2.5.

In another instance, the Rab6a:Rab GGTase molar ratio is about 1:0.25-0.75, 1:0.3-0.7, 1:0.35-0.65, 1:0.4-0.6 or 1:0.45-0.55, preferably about 1:0.4-0.6. In one instance, the Rab6a:Rab GGTase molar ratio is about 1:0.25, 1:0.3, 1:0.35, 1:0.4, 1:0.45, 1:0.5, 1:0.55, 1:0.6, 1:0.65, 1-0.7 or 1:0.75, preferably about 1:0.5.

In one instance, the lipid donor substrate is geranylgeranylpyrophosphate (GGPP) or an analogue thereof. Preferably, the lipid donor substrate is labelled with a detectable marker. For example, the lipid donor substrate may be isotopically labelled (e.g. the lipid donor substrate may comprise ³H), or may comprise a fluorescent group, epitope or biotin moiety.

In a preferred instance, the lipid donor substrate is biotin-geranylpyrophosphate (BGPP).

In one instance, the lipidated Rab6a product is detected using an enzyme-linked immunosorbent assay (ELISA), Western blot analysis or autoradiography. In a preferred instance, the lipidated Rab6a product is detected using an ELISA. The ELISA may be, for example, a sandwich ELISA.

In a preferred instance, a biotin-labelled lipidated Rab6a product is detected using a detection reagent specific for biotin, for example streptavidin. Preferably, the biotin-labelled lipidated Rab6a product is detected using Western blot analysis using a detection reagent specific for biotin, for example streptavidin (e.g. a streptavidin-horseradish peroxidase conjugate). More preferably, the biotin-labelled lipidated Rab6a product is detected using an ELISA using a detection reagent specific for biotin, for example streptavidin.

In one instance, the method is for determining the activity of a REP1-encoding gene therapy vector for use in the treatment of choroideremia.

In another aspect, the disclosure provides the use of Rab6a for determining the activity of Rab escort protein 1 (REP1).

The method of determining the activity of REP1, the Rab6a, Rab GGTase, lipid donor substrate and the REP1 may be as described herein.

In another aspect, the disclosure provides a method for determining the efficacy of a vector comprising a Rab escort protein 1 (REP1) encoding nucleotide sequence, wherein the method comprises the steps:
(a) providing a sample comprising REP1, wherein the REP1 is expressed using the vector comprising a REP1-encoding nucleotide sequence;
(b) contacting the sample of step (a) with Rab6a, Rab geranylgeranyltransferase (Rab GGTase) and a lipid donor substrate; and
(c) detecting the lipidated Rab6a product.

In another aspect, the disclosure provides the use of Rab6a for determining the efficacy of a vector comprising a Rab escort protein 1 (REP1) encoding nucleotide sequence.

Preferably, the method and use are for determining the efficacy of a vector for use in the treatment of choroideremia.

The vector, REP1, Rab6a, Rab GGTase, lipid donor substrate, lipidated Rab6a product, method of detection and other features of the method and use may be as described herein.

### DESCRIPTION OF THE DRAWINGS

**Figure 1**
   **In vitro prenylation reaction using Rab27a as a substrate followed by Western blot (WB) analysis of incorporated biotinylated lipid donor using untransduced cells and cells transduced with either AAV-GFP or AAV-REP1 (MOI = 10000 gp/cell).**
   The experiment involved 3 sets of lysates prepared independently. Prenylation reactions were set up using 10 µg of lysate in a total volume of 12.5 µL. Positive controls were spiked with 2 µM of fish REP1. Detection time was 2 min.
   (A) WB analysis of human REP1 levels in cell lysates (1:1000). Untransduced cells (#4, #9 and #12) show endogenous levels of REP1. Cells transduced with AAV-GFP (#5, #10 and #13) show endogenous levels of REP1 similar to untransduced cells. Cells transduced with AAV-REP1 (#6, #11 and #14) show an increase of REP1 levels compared to untransduced and AAV-GFP transduced cells. Positive controls (+ve) show endogenous REP1 levels.
   (B) WB analysis of β-actin as loading control (1:15000). The levels of β-actin are similar in all samples analysed.
   (C) WB analysis of incorporated biotinylated lipid donor in Rab27a (1:10000). Untransduced cells and AAV-GFP transduced cells show no detectable incorporation of biotin in Rab27a. Cells transduced with AAV-REP1 show some level of biotin incorporated into Rab27a (#6, #11 and #14). Positive controls show the strongest band of all as a result of fish REP1 activity.
   **(D)** Semiquantification of the WB analysis in (C) using Image Studio Lite software.
**Figure 2**
   **In vitro prenylation reaction using Rab27a as a substrate followed by Western blot (WB) analysis of incorporated biotinylated lipid donor using untransduced cells and cells transduced with either AAV-GFP or AAV-REP1 (MOI = 10000 gp/cell).**
   The experiment involved 2 sets of lysates prepared independently. Prenylation reactions were set up using 30 µg of lysate in a total volume of 22 µL. Positive controls were spiked with 1 µM of fish REP1. Detection time was 2 min.
   **(A)** WB analysis of human REP1 levels in cell lysates (1:1000). Untransduced cells (#9 and #12) show endogenous levels of REP1. Cells transduced with AAV-GFP (#10 and #13) show endogenous levels of REP1 similar to untransduced cells. Cells transduced with AAV-REP1 (#11 and #14) show an increase of REP1 levels compared to untransduced and AAV-GFP transduced cells. Positive controls (+ve) show endogenous REP1 levels.
   **(B)** WB analysis of β-actin as loading control (1:15000). The levels of β-actin are similar in all samples analysed.
   **(C)** WB analysis of incorporated biotinylated lipid donor in Rab27a (1:10000). Untransduced cells and AAV-GFP transduced cells show no detectable incorporation of biotin in Rab27a. Cells transduced with AAV-REP1 show some level of biotin incorporated into Rab27a (#11 and #14). Positive controls show the strongest band of all as a result of fish REP1 activity.
   **(D)** Semiquantification of the WB analysis in (C) using Image Studio Lite software.
**Figure 3**
   **In vitro prenylation reaction using Rab6a as a substrate followed by Western blot (WB) analysis of incorporated biotinylated lipid donor using untransduced cells and cells transduced with either AAV-GFP or AAV-REP1 (MOI = 10000 gp/cell).**
   The experiment involved 2 sets of lysates prepared independently. Prenylation reactions were set up using 20 µg of lysate in a total volume of 20 µL. Positive controls were spiked with 1 µM of fish REP1. Detection time was 2 min.
   **(A)** WB analysis of human REP1 levels in cell lysates (1:1000). Untransduced cells (#9 and #12) show endogenous levels of REP1. Cells transduced with AAV-GFP (#10 and #13) show endogenous levels of REP1 similar to untransduced cells. Cells transduced with AAV-REP1 (#11 and #14) show an increase of REP1 levels compared to untransduced and AAV-GFP transduced cells. Positive controls (+ve) show endogenous REP1 levels.
   **(B)** WB analysis of β-actin as loading control (1:15000). The levels of β-actin are similar in all samples analysed.
   **(C)** WB analysis of incorporated biotinylated lipid donor in Rab6a (1:10000). Untransduced cells and AAV-GFP transduced cells show very low incorporation of biotin in Rab6a. Cells transduced with AAV-REP1 show biotin incorporated into Rab6a (#11 and #14). Positive controls show the strongest band of all as a result of fish REP1 activity.
   **(D)** Semiquantification of the WB analysis in (C) using Image Studio Lite software.
**Figure 4**
   **In vitro prenylation reaction using Rab6a as a substrate followed by Western blot (WB) analysis of incorporated biotinylated lipid donor using untransduced cells and cells transduced with AAV-REP1 (MOI = 250, 1000, 5000, 10000 and 20000 gp/cell).**
   Prenylation reactions were set up using 20 µg of lysate in a total volume of 15 µL. The positive control was spiked with 0.5 µM of fish REP1. Detection time was 2 min.
   **(A)** WB analysis of human REP1 levels in cell lysates (1:2500). Untransduced cells show endogenous levels of REP1. Cells transduced with AAV-REP1 show an increase of REP1 levels that directly correlates with the MOI used. The positive control (+ve) shows endogenous REP1 levels.
   **(B)** WB analysis of β-actin as loading control (1:50000). The levels of β-actin are similar in all samples analysed.
   **(C)** WB analysis of incorporated biotinylated lipid donor in Rab6a (1:10000). Untransduced cells show very low of biotin incorporation in Rab6a. Cells transduced with AAV-REP1 show increasingly more biotin incorporated into Rab6a. The positive control shows the strongest band of all as a result of fish REP1 activity.
   **(D)** Semiquantification of the WB analysis in (C) using Image Studio Lite software.
**Figure 5**
   **In vitro prenylation reaction using Rab6a as a substrate followed by Western blot (WB) analysis of incorporated biotinylated lipid donor using untransduced cells and cells transduced with AAV-REP1 (MOI = 10000 gp/cell).**
   Prenylation reactions were set up using 20 µg of lysate in a total volume of 15 µL. The positive control was spiked with 0.5 µM of fish REP1. Detection time was 2 min for REP1/actin and 30 seconds for biotin.
   **(A)** WB analysis of human REP1 levels in cell lysates (1:2500). Untransduced cells show endogenous level of REP1. Cells transduced with AAV-REP1 show an increase of REP1 levels. The positive control (+ve) shows endogenous REP1 levels.
   **(B)** WB analysis of β-actin as loading control (1:50000). The levels of β-actin are similar in all samples analysed.
   **(C)** WB analysis of incorporated biotinylated lipid donor in Rab6a (1:10000). Untransduced cells show very low biotin incorporation in Rab6a. Cells transduced with AAV-REP1 show increased biotin incorporation into Rab6a. The positive control shows the strongest band of all as a result of fish REP1 activity.
   **(D)** Semiquantification of WB analysis in (C) using Image Studio Lite software.
**Figure 6**
   **In vitro prenylation reaction, followed by Western blot (WB) analysis of incorporated biotinylated lipid donor in untransduced ARPE-19 cells, and ARPE-19 cells transduced with AAV-REP1.**
   Prenylation reactions were set up using 15 µg of lysate in a total volume of 45 µL. Positive control was spiked with 0.1 µM of fish REP1. Detection time for REP1/actin: 2 min; for biotin: 30 seconds.
   Experiments were carried out in parallel using the following cells:
   (a) Untransduced cells (#86 and #87); and
   (b) Cells + AAV-REP1 MOI 10,000 (#90 and #91) - R&D grade vector.
      **(A)** WB analysis of human REP1 levels in cell lysates (1:2,500). Untransduced cells show endogenous level of REP1. Cells transduced with AAV-REP1 show an increase of REP1 levels. Positive control (+ve) shows endogenous REP1 levels.
      **(B)** WB analysis of β-actin as loading control (1:50,000). The levels of β-actin are similar in all samples analysed.
      **(C)** WB analysis of incorporated biotinylated lipid donor in Rab6a (1:10,000). Untransduced cells show very low incorporation of biotin in Rab6a; cells transduced with AAV-REP1 show increased biotin incorporation into Rab6a. Positive control shows the strongest band of all, as a result of fish REP1 activity.
      **(D)** Semiquantification of WB analysis in (C) using Image Studio Lite software.
**Figure 7**
   **In vitro prenylation reaction, followed by Western blot (WB) analysis of incorporated biotinylated lipid donor in untransduced HT1080 cells, and HT1080 cells transduced with AAV-REP1.**
   Prenylation reactions were set up using 20 µg of lysate in a total volume of 20 µL. Positive control was spiked with 0.1 µM of fish REP1. Detection time for REP1/actin: 2 min; for biotin: 30 seconds.
   Experiments were carried out in parallel using the following cells:
   (a) Untransduced cells (#56 and #57);
   (b) Cells + AAV-REP1 MOI 10,000 (#60 and #61) - R&D grade vector; and
   (c) Cells + AAV-REP1 MOI 10,000 (#64 and #65) - clinical grade vector.
      **(A)** WB analysis of human REP1 levels in cell lysates (1:2,500). Untransduced cells show endogenous levels of REP1. Cells transduced with AAV-REP1 show an increase of REP1 levels. Positive control (+ve) shows endogenous REP1 levels.
      **(B)** WB analysis of β-actin as loading control (1:50,000). The levels of β-actin are similar in all samples analysed.
      **(C)** WB analysis of incorporated biotinylated lipid donor in Rab6a (1:10,000). Untransduced cells show baseline levels of biotin incorporation in Rab6a; cells transduced with AAV-REP1 show increased biotin incorporation into Rab6a.
      **(D)** Semiquantification of WB analysis in (C) using Image Studio Lite software.
**Figure 8**
   **In vitro prenylation reaction, followed by Western blot (WB) analysis of incorporated biotinylated lipid donor in untransduced cells, and cells transduced with AAV-REP1 (MOI = 250, 1000, 5000, 10000 and 20000 gp/cell) comparing Rab6a and Rab27a substrates.**
   Prenylation reactions were set up using 20 µg of lysate in a total volume of 15 µL, and 2 different substrates: Rab27a (left-hand lanes and plots; in red) and Rab6a (right-hand lanes and plots; in blue). Positive controls, one for each substrate, were spiked with 0.1 µM of fish REP1. Detection time: 2 min.
   **(A)** WB analysis of human REP1 levels in cell lysates (1:2,500). Untransduced cells show endogenous level of REP1. Cells transduced with AAV-REP1 show an increase of REP1 levels that directly correlates with the MOI used. Positive control (+ve) shows endogenous REP1 levels.
   **(B)** WB analysis of β-actin as loading control (1:50,000). The levels of β-actin are similar in all samples analysed.
   **(C)** WB analysis of incorporated biotinylated lipid donor in Rab27a and Rab6a. Untransduced cells show very low incorporation of biotin into the Rab protein, which increases as the MOI increases. Positive controls show strong biotin incorporation, as a result of fish REP1 activity.
   **(D)** Semiquantification of WB analysis in (C) using Image Studio Lite software. Data was plotted using Prism software as shown in the four plots in (E).
   **(E)** Band density values from biotinylated substrates across MOIs used (upper two plots) and ratio between biotinylated substrates and REP1 across MOIs used (lower two plots).
**Figure 9**
   **In vitro prenylation reaction, followed by Western blot (WB) analysis of incorporated biotinylated lipid donor in untransduced cells comparing different prenylation reaction conditions.**
   Prenylation reactions were set up using 20 µg of lysate in a total volume of 15 µL, and 2 different substrates: Rab27a (in red) and Rab6a (in blue). Positive controls, one for each substrate, were spiked with recombinant human REP1. Detection time: 2 min.
   **(A)** WB analysis of incorporated biotinylated lipid donor in Rab27a and Rab6a. Level of biotin incorporation is directly proportional to the amount of total protein in the reaction. Positive controls show strong biotin incorporation, as a result of fish REP1 activity.
   **(B)** WB analysis of β-actin as loading control (1:50,000). The levels of β-actin match the amount of total cell lysates used in the reaction, and are similar between samples.
   **(C)** WB analysis of human REP1 levels in cell lysates (1:2,500). Untransduced cells show endogenous level of REP1. Positive control (+ve) shows higher density of REP1.
   **(D)** Semiquantification of WB analysis in (A) using Image Studio Lite software. Data was plotted using Prism software. Values highlighted are for those conditions where a higher difference between substrates was detected.
**Figure 10**
   **Comparison between Rab27a and Rab6a as substrates for prenylation in AAV-REP1 transduced cells.**

### DETAILED DESCRIPTION

Various preferred features and instances of the present disclosure will now be described by way of non-limiting examples.

The practice of the present disclosure will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

### Prenylation

Lipidation of proteins by the addition of isoprenoid moieties is a post-translational modification that affects up to 2% of the mammalian proteome. Such lipidation enables reversible association of the target proteins with cell membranes and can also modulate protein-protein interactions.

Preferably, the lipidation referred to herein is prenylation, such that the lipid donor substrate and lipidated Rab6a product are a prenyl donor substrate and prenylated Rab6a product, respectively.

Prenylation is a specific type of post-translational modification in which a geranylgeranyl or farnesyl moiety (or analogue of either) is attached to one or two C-terminal cysteine residues of a protein via a thioether linkage.

Preferably, the prenylation is the addition of a geranylgeranyl moiety or an analogue thereof (e.g. biotin-geranyl moiety) to a target protein (e.g. Rab6a).

A geranylgeranyl moiety attached to a protein (the protein is depicted schematically by the shaded circle) is:

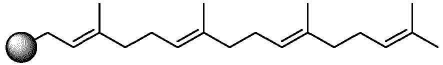

A farnesyl moiety attached to a protein (the protein is depicted schematically by the shaded circle) is:

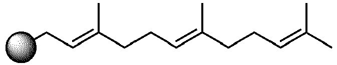

The term "analogue" is used herein in relation to the lipid (e.g. geranylgeranyl or farnesyl) moiety or lipid donor substrate to refer to a compound which has been modified to comprise a functional group suitable for a particular purpose, such as detection. The analogue is able to be added to a substrate protein by the prenylation machinery (i.e. REP1 and Rab GGTase) in a manner substantially unhindered (for the purposes of the activity assays of the disclosure) by the modification.

Accordingly, analogues of the above moieties include those which have been artificially created for particular purposes (e.g. labelled moieties which are suitable for detection in an assay). In particular, Nguyen et al. (Nguyen, U.T. et al. (2009) Nat. Chem. Biol. 5: 227-235) developed the following biotin-geranyl moiety that can be detected in in vitro protein prenylation reactions (the biotin-geranyl moiety is shown attached to a protein, which is depicted schematically by the shaded circle):

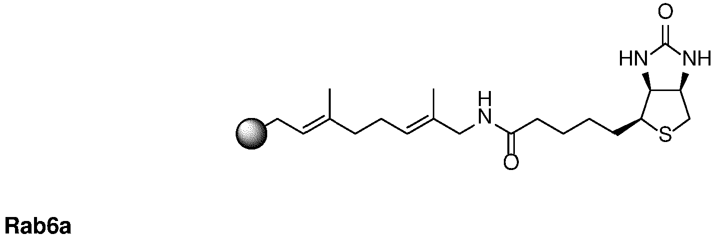

Rab6a (Ras-related protein Rab-6A) is a member of the mammalian Rab GTPase family, which is itself the largest of the Ras-like super-family of GTPases.

Rab GTPases (also known as Rab proteins) are peripheral membrane proteins and are involved in the regulation of membrane trafficking, including vesicle formation, vesicle movement along actin and tubulin networks, and membrane fusion. The main function of Rab6a is understood to be the regulation of protein transport from the Golgi complex to the endoplasmic reticulum.

Rab GTPases are typically anchored to a cell membrane via prenyl groups (in particular, geranylgeranyl groups) which are covalently bound to two C-terminal cysteine residues.

Rab GTPases exhibit two conformations: an inactive, GDP-bound form; and an active, GTP-bound form. Conversion from the GDP- to the GTP-bound forms is catalysed by a GDP/GTP exchange factor (GEF), which thereby activates the Rab GTPase. Conversely, GTP hydrolysis by Rab GTPases can be enhanced by a GTPase-activating protein (GAP), which thereby leads to Rab inactivation.

In one instance, the Rab6a is human Rab6a.

An example amino acid sequence of Rab6a is the sequence deposited under NCBI Accession No. NP_942599.1.

An example amino acid sequence of Rab6a is:

An example nucleotide sequence encoding Rab6a is the sequence deposited under NCBI Accession No. NM_198896.1.

An example nucleotide sequence encoding Rab6a is:

### Rab geranylgeranyltransferase (Rab GGTase)

Rab geranylgeranyltransferase (Rab GGTase; also known as geranylgeranyltransferase II) is a protein prenyltransferase which exclusively prenylates the GTPases of the Rab family.

Rab GGTase typically naturally catalyses the transfer of two geranylgeranyl groups to cysteine residues at the C-terminus of Rab GTPases. Each geranylgeranyl group is conjugated to the Rab GTPase via a thioether linkage to a cysteine residue.

Rab GGTase has been shown to be capable of binding a range of derivatised phosphoisoprenoids and can catalyse their addition to Rab GTPase substrates (e.g. Rab6a). For example, Nguyen et al. (Nguyen, U.T. et al. (2009) Nat. Chem. Biol. 5: 227-235) demonstrated the successful addition of a biotin-geranyl moiety to Rab GTPases.

Rab GGTase is a heterodimeric enzyme comprised of alpha and beta subunits.

In one instance, the Rab GGTase is human Rab GGTase. In a preferred instance, the Rab GGTase is rat Rab GGTase.

Example amino acid sequences of Rab GGTase alpha subunits are the sequences deposited under NCBI Accession Nos. NP_004572.3 and NP_113842.1.

Example amino acid sequences of Rab GGTase alpha subunits are: and:

Example amino acid sequences of Rab GGTase beta subunits are the sequences deposited under NCBI Accession Nos. NP_004573.2. and NP_619715.1.

Example amino acid sequences of Rab GGTase beta subunits are: and:

### Lipid donor substrate

To add a lipid moiety to a Rab GTPase, the Rab GGTase may use the lipid moiety in the form of a lipid (e.g. geranylgeranyl or biotin-geranyl) donor substrate as a substrate. These are typically pyrophosphate derivatives of the lipid moiety.

For example, geranylgeranylpyrophosphate (GGPP) or biotin-geranylpyrophosphate (BGPP) may be used as lipid donor substrates by Rab GGTase to transfer a geranylgeranyl or biotin-geranyl moiety, respectively, to the substrate Rab GTPase.

Geranylgeranylpyrophosphate has the structure:

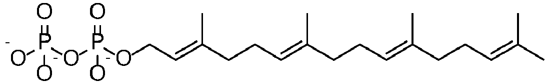

An example structure of biotin-geranylpyrophosphate is:

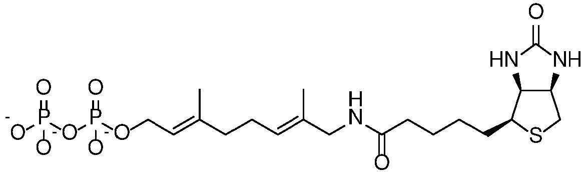

### Rab escort protein 1 (REP1)

Rab escort proteins (REPs) perform the functions of presenting unprenylated Rab GTPases to Rab GGTases, and carrying prenylated Rab GTPases to their target membranes.

Rab GTPases do not comprise a consensus sequence at the prenylation site that may be recognised by Rab GGTases. However, substrate recognition is effected through REPs, which bind Rab GTPases through a conserved region and then present the Rab GGTase with its substrate for prenylation.

Once prenylated, the lipid anchors render the Rab GTPases insoluble. Accordingly, REPs are required to bind and solubilise the geranylgeranyl groups and aid delivery of the Rab GTPase to the target cell membrane.

REP1 may also be known as Rab protein geranylgeranyltransferase component A. Furthermore, the gene that encodes REP1 may be known as the CHM gene.

In one instance, the REP1 is human REP1.

An example amino acid sequence of REP1 is:

An example nucleotide sequence encoding REP1 is:

A further example nucleotide sequence encoding REP1 is:

Example variants of REP1 are described further in WO 2012/114090.

### Activity determination

In one aspect, the disclosure provides a method for determining the activity of Rab escort protein 1 (REP1) comprising the steps:
(a) providing a sample comprising REP1;
(b) contacting the sample of step (a) with Rab6a, Rab geranylgeranyltransferase (Rab GGTase) and a lipid donor substrate; and
(c) detecting the lipidated Rab6a product.

In another aspect, the disclosure provides the use of Rab6a for determining the activity of Rab escort protein 1 (REP1).

Assay sensitivity is an important factor to consider, because it enables detection of low levels of a target, which is particularly relevant when small quantities of reagents are present (e.g. as may be the case with gene therapy reagents). However, it is also important to maximise the dynamic range of an assay's signal, which may, for example, not correlate with reagents that provide low or high sensitivity.

The method and use of the disclosure are for testing the activity of REP1, rather than testing other agents that are involved in the prenylation of a Rab GTPase, for example, the activity of Rab GGTases or lipid donor substrates, or the activity of Rab GTPases as prenylation substrates. For example, the method of the disclosure may be for testing gene therapy vectors suitable for the delivery of REP1 to a target cell or for quality control analysis of vector stocks (e.g. medicament stocks).

In one instance, the sample comprising REP1 is from a cell genetically engineered to express the REP1. Preferably, a cell is transfected or transduced with a vector comprising a REP1-encoding nucleotide sequence to provide the cell genetically engineered to express the REP1. Preferably, the vector is a viral vector.

In one instance, the REP1 is expressed using a viral vector comprising a REP1-encoding nucleotide sequence.

The cell (which may be as a population of such cells) which is genetically engineered to express the REP1 may be any cell suitable for genetic engineering and expression of REP1, such as a cell from a cell line (e.g. HEK293). The cell may be, for example, a human or mouse cell. Preferably, the cell is a human cell. The cell may, for example, be a retinal cell, such as a retinal pigment epithelial or photoreceptor cell. In one instance, the cell is a HEK293 cell. In another instance, the cell is an ARPE-19 cell. In another instance, the cell is an HT1080 cell.

Preferably, the Rab6a and/or Rab GGTase are from a standard source such that they provide for minimal or no variation in repeated experiments. Preferably, the Rab6a and/or Rab GGTase are substantially pure (i.e. comprise substantially no protein contaminants that interfere with the method or use of the disclosure).

Accordingly, the method or use may comprise carrying out a plurality of experiments (e.g. comprising steps (a) to (c)) in which parameters relating to the sample comprising REP1 are varied, while other parameters (e.g. parameters relating to the Rab6a, Rab GGTase and lipid donor substrate) are kept constant. Such parameters may include, for example, the amino acid sequence of the relevant protein (e.g. REP1), the REP1-encoding nucleotide sequence comprised in a vector used to express the REP1 in a cell, the type of vector used to deliver a REP1-encoding nucleotide sequence to a cell (e.g. the type of viral vector, such as the type of adeno-associated viral (AAV) vector), the concentration of REP1 and/or the multiplicity-of-infection (MOI) of a vector used to deliver a REP1-encoding nucleotide sequence to a cell.

The term "activity" is used herein to refer to the ability of REP1 to facilitate the lipidation of a Rab GTPase (e.g. Rab6a). Although the REP1 does not catalyse the lipidation itself, it is required for a Rab GGTase to catalyse the lipidation of its substrate Rab GTPase. Accordingly, the activity of the REP1 may be measured by determining the amount of Rab GTPase (i.e. Rab6a) which is lipidated under certain conditions.

The term "efficacy" is used herein, in relation to efficacy of a vector comprising a REP1-encoding nucleotide sequence, to refer to the ability of the vector to provide REP1 activity to a cell which is transfected or transduced by the vector.

The term "lipidated Rab6a product" as used herein refers to Rab6a to which a lipid moiety has been added. Preferably, the lipidated Rab6a product is a prenylated Rab6a, such as a geranylgeranylated Rab6a or a biotin-geranylated Rab6a.

Preferably, the step of detecting the lipidated Rab6a product provides quantification of the amount of lipidated Rab6a product.

The detection of lipidated Rab6a may be carried out by any suitable method, for example an enzyme-linked immunosorbent assay (ELISA), a Western blot, autoradiography (e.g. utilising an isotopically-labelled, such as tritiated, lipid donor substrate), chromatographic (e.g. HPLC or FPLC) and/or mass spectrometry-based method (e.g. LC/MS).

In one instance, the lipidated Rab6a product is detected using a Western blot. In a preferred instance, the lipidated Rab6a product is detected using an ELISA.

By way of example, a prenylation reaction may be carried out according to the method of the disclosure using a biotin-geranylpyrophosphate lipid donor substrate. The product of the reaction may be subjected to Western blot analysis in which the lipidated Rab6a product (i.e. biotin-geranylated Rab6a) may be detected by direct incubation with, for example, streptavidin-horseradish peroxidase conjugate. Quantification of the lipidated Rab6a (i.e. biotin-geranylated Rab6a) may be achieved by densitometric analysis of the resulting Western blot, which may be carried out by any suitable means (e.g. using Image Studio Lite software (LI-COR)).

By way of further example, a prenylation reaction may be carried out according to the method of the disclosure using a biotin-geranylpyrophosphate lipid donor substrate. The product of the reaction may be subjected to an ELISA analysis in which the Rab6a may be immobilised on a plate directly or using an antibody that has been attached to the plate (i.e. a sandwich ELISA); and then the lipidated Rab6a product (i.e. biotin-geranylated Rab6a) may be detected by incubation with, for example, streptavidin-horseradish peroxidase conjugate. Quantification of the lipidated Rab6a (i.e. biotin-geranylated Rab6a) may be achieved by any suitable means (e.g. detection using a spectrophotometer, fluorometer or luminometer).

Further detection steps may be incorporated into the method of the disclosure, as required (e.g. for control purposes), such as the detection of the amount of REP1 present in the reaction or detection of the amount of β-actin (e.g. as a loading control).

In one instance, the method comprises a further step of comparing the amount of lipidated Rab6a product (e.g. prenylated, such as geranylgeranylated or biotin-geranylated, Rab6a) with an amount determined from a control experiment, such as an experiment using a known or standard sample of REP1.

In another instance, the method comprises a further step of comparing the amount of lipidated Rab6a product (e.g. prenylated, such as geranylgeranylated or biotin-geranylated, Rab6a) with a reference level.

Comparison with such control experiments or reference levels may provide a measure of the activity of the REP1 relative to a known or accepted standard (e.g. better or worse than a known or accepted standard).

The method of the disclosure may, for example, be used for quality control analysis of a gene therapy vector for the treatment of choroideremia, preferably an AAV vector particle comprising a REP1-encoding nucleotide sequence, wherein an output activity or efficacy of the vector determined by the method of the disclosure above a threshold activity or within a specified target range (e.g. by comparison to a control experiment or reference level) indicates the vector is suitable for gene therapy purposes.

The conditions of the prenylation reaction (e.g. that occurring in step (b) of the method of the disclosure) are not particularly limited, providing that they do not substantially interfere with the prenylation of Rab6a.

The sample comprising REP1 may be formulated in any suitable form, for example the sample may be prepared in a prenylation buffer comprising about 50 mM HEPES, 50 mM NaCl, 2 mM MgCl₂, 1 mM DTT and protease inhibitor cocktail (Roche) at about pH 7.5.

The sample comprising REP1 may, for example, comprise about 1-100, 1-75, 1-50, 1-40, 1-30, 1-20 or 1-10 µg of total protein. The sample comprising REP1 may, for example, comprise about 10-100, 10-75, 10-50, 10-40, 10-30 or 10-20 µg of total protein. Preferably, the sample comprising REP1 comprises about 10-30 µg of total protein, for example, about 10, 15, 20, 25 or 30 µg of total protein.

The Rab6a may, for example, be at a concentration of about 1-25, 1-20, 1-15, 1-10 or 1-5 µM, preferably about 1-5 µM. The Rab6a may, for example, be at a concentration of about 1, 2, 3, 4, 5, 6, 7, 8, 9, 10, 11, 12, 13, 14, 15, 16, 17, 18, 19, 20, 21, 22, 23, 24 or 25 µM, preferably about 4 µM.

The Rab GGTase may, for example, be at a concentration of about 1-25, 1-20, 1-15, 1-10, 1-5 or 1-2.5 µM, preferably about 1-2.5 µM. The Rab GGTase may, for example, be at a concentration of about 1, 2, 3, 4, 5, 6, 7, 8, 9, 10, 11, 12, 13, 14, 15, 16, 17, 18, 19, 20, 21, 22, 23, 24 or 25 µM, preferably about 2 µM.

The lipid donor substrate (e.g. biotin-geranylpyrophosphate (BGPP)) may, for example, be at a concentration of about 1-25, 1-20, 1-15, 1-10 or 1-5 µM, preferably about 1-5 µM. The lipid donor substrate (e.g. biotin-geranylpyrophosphate (BGPP)) may, for example, be at a concentration of about 1, 2, 3, 4, 5, 6, 7, 8, 9, 10, 11, 12, 13, 14, 15, 16, 17, 18, 19, 20, 21, 22, 23, 24 or 25 µM, preferably about 4 µM.

The prenylation reaction may be carried out in any suitable buffer, for example the reaction may be carried out in a prenylation buffer comprising about 50 mM HEPES, 50 mM NaCl, 2 mM MgCl₂, 1 mM DTT and protease inhibitor cocktail (Roche) at about pH 7.5.

Prenylation reactions may be carried out for any suitable length of time at any suitable temperature (e.g. about 37°C). For example, prenylation reactions may be carried out for about 1-10, 1-7.5, 1-5, 1-2.5 or 1-2 h, preferably about 1-2 h. Prenylation reactions may, for example, be carried out for about 1, 2, 3, 4, 5, 6, 7, 8, 9 or 10 h, preferably about 2 h.

### Choroideremia

Choroideremia is a rare X-linked progressive degeneration of the choroid, retinal pigment epithelium and photoreceptors of the eye. The typical natural history in afflicted males is onset of nightblindness during teenage years, and then progressive loss of peripheral vision during the 20's and 30's leading to complete blindness in the 40's. Female carriers have mild symptoms, most notably nightblindness, but may occasionally have a more severe phenotype.

Choroideremia is caused by mutations in the *CHM* gene, which is located on the X chromosome 21q region. Rab escort protein 2 (REP2), which is 75% homologous to REP1, compensates for any REP1 deficiency in most cells of the body. However, for reasons that are not yet clear, REP2 is unable to compensate for REP1 deficiency in the eye. This leads to insufficient Rab escort protein activity to maintain normal prenylation of target Rab GTPases and gives rise to cellular dysfunction and ultimately cell death, primarily affecting the outer retina and choroid.

Choroideremia may be successfully treated by providing functional copies of the REP1 transgene to the affected cells of the eye (MacLaren, R.E. et al. (2014) Lancet 383: 1129-37).

### Vectors

A vector is a tool that allows or facilitates the transfer of an entity from one environment to another. In accordance with the disclosure, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (e.g. DNA or RNA) within the cell, facilitating the replication of the vector comprising a segment of nucleic acid or facilitating the expression of the protein encoded by a segment of nucleic acid.

Vectors may be non-viral or viral. Examples of vectors used in recombinant nucleic acid techniques include, but are not limited to, plasmids, chromosomes, artificial chromosomes and viruses. The vector may also be, for example, a naked nucleic acid (e.g. DNA or RNA). In its simplest form, the vector may itself be a nucleotide of interest.

The vectors used in the disclosure may be, for example, plasmid or viral vectors and may include a promoter for the expression of a polynucleotide and optionally a regulator of the promoter.

### Viral Vectors

In a preferred instance, the vector of the disclosure is a viral vector. Preferably, the viral vector is in the form of a viral vector particle.

The viral vector may be, for example, an adeno-associated viral (AAV), retroviral, lentiviral or adenoviral vector. Preferably, the viral vector is an AAV vector.

The term "gene therapy vector" is used herein to refer to a vector which is suitable for use in gene therapy and includes, for example, viral (e.g. AAV) vectors and vector particles.

### Variants, derivatives, analogues, homologues and fragments

In addition to the specific proteins and nucleotides mentioned herein, the disclosure also encompasses the use of variants, derivatives, analogues, homologues and fragments thereof.

In the context of the disclosure, a variant of any given sequence is a sequence in which the specific sequence of residues (whether amino acid or nucleic acid residues) has been modified in such a manner that the polypeptide or polynucleotide in question substantially retains its function. A variant sequence can be obtained by addition, deletion, substitution, modification, replacement and/or variation of at least one residue present in the naturally-occurring protein.

The term "derivative" as used herein, in relation to proteins or polypeptides of the disclosure includes any substitution of, variation of, modification of, replacement of, deletion of and/or addition of one (or more) amino acid residues from or to the sequence providing that the resultant protein or polypeptide substantially retains at least one of its endogenous functions.

The term "analogue" as used herein, in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions provided that the modified sequence substantially retains the required activity or ability. Amino acid substitutions may include the use of non-naturally occurring analogues.

Proteins used in the disclosure may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" as used herein means an entity having a certain homology with the wild type amino acid sequence and the wild type nucleotide sequence. The term "homology" can be equated with "identity".

A homologous sequence may include an amino acid sequence which may be at least 50%, 55%, 60%, 65%, 70%, 75%, 80%, 85% or 90% identical, preferably at least 95% or 97% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the disclosure it is preferred to express homology in terms of sequence identity.

A homologous sequence may include a nucleotide sequence which may be at least 50%, 55%, 60%, 65%, 70%, 75%, 80%, 85% or 90% identical, preferably at least 95% or 97% or 99% identical to the subject sequence. Although homology can also be considered in terms of similarity, in the context of the disclosure it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye or, more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; and FEMS Microbiol. Lett. (1999) 177: 187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

"Fragments" of full length polypeptides or polynucleotides of the disclosure are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the disclosure to make the encoded protein. These methods are only illustrative of the numerous standard techniques known in the art for manipulation of DNA sequences and other known techniques may also be used.

### Codon optimisation

The polynucleotides used in the present disclosure may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

### EXAMPLES

### Materials and methods (Examples 1-2)

### Cell transduction and harvesting

Cultured HEK293 cells were treated with rAAV2/2-REP1 at a range of multiplicities of infection (MOI, genome particles/cell). rAAV2/2-GFP was used in parallel as a control vector, and fluorescence was monitored for onset of transgene expression.

Experiments on untransduced, and +AAV-GFP transduced and +AAV-REP1 transduced cells were conducted in parallel.

Cell lysates were prepared at day 5 post-transduction using the following protocol: cells were washed with PBS and incubated for 5 min with prenylation buffer, pH 7.5 (50 mM HEPES, 50 mM NaCl, 2 mM MgCl₂, 1 mM DTT and protease inhibitor cocktail (Roche)) on ice; cells were then scraped using a cell scraper into a 1.5 mL tube and incubated on ice for 15 min; subsequently, cells were disrupted by pushing them 20 times through a 26-G syringe needle attached to a 1 mL syringe.

Lysed cells were centrifuged for 5 min at 1500xg at 4°C. The supernatant was then transferred to cellulose propionate tubes and centrifuged for 1 h at 100000xg at 4°C. The supernatant from the second centrifugation step was used for the in vitro prenylation reactions (described below).

### Total protein quantification

Total cell protein concentration was quantified using the Bradford method according to the manufacturer's instructions (Quick Start™ Bradford 1x Dye Reagent, BioRad, #500-0205). Sample values were extrapolated from a standard curve.

### In vitro prenylation reaction

Prenylation reactions were set up using frozen cell lysate (10-30 µg), 2 µM Rab GGTase, 4 µM Rab protein (Rab27a or Rab6a) and 5 µM biotin-geranylpyrophosphate (BGPP) as the lipid donor, in prenylation buffer. All reactions were supplemented with fresh GDP (guanosine diphosphate, 20 µM) and DTT (1 mM).

For positive control samples, fish REP1 (see individual experiments for the amount) was added to the prenylation reaction containing lysate from untransduced cells.

Reactions were incubated for 2 h at 37°C and then stopped by addition of sample buffer (Laemmli buffer, 2× concentrate, Sigma #S3401). This buffer contains 4% SDS, 20% glycerol, 10% 2-mercaptoethanol, 0.004% bromphenol blue and 0.125 M Tris HCI, pH approx. 6.8.

Western blots (WB) were performed to detect human REP-1, β-actin (as a loading control) and biotinylated Rab protein (Rab27a or Rab6a).

For detection of human REP1, a mouse monoclonal antibody from Millipore was used (clone 2F1, #MABN52). For detection of β-actin, a mouse monoclonal antibody from Thermo Fisher Scientific was used (clone AC-15, #AM4302). Both detections were followed by a secondary antibody-labelling step (donkey anti-mouse HRP, Abcam, #ab98799).

The incorporation of biotinylated lipid donor into the appropriate Rab substrate was detected by direct incubation with streptavidin-HRP (Thermo Fisher Scientific, #43-4323).

All membranes were detected using ECL substrate and Odyssey FC detection system (LI-COR). The intensities of the bands were quantitatively analysed using Image Studio Lite software (LI-COR).

### Example 1 - Rab27a as a prenylation substrate

To test the sensitivity of a prenylation assay using Rab27a as a substrate, experiments were carried out in parallel using the following cells:
(a) untransduced cells;
(b) cells transduced with AAV-GFP at a MOI of 10000; and
(c) cells transduced with AAV-REP1 at a MOI of 10000.

Prenylation reactions were set up using 10 µg of lysate in a total volume of 12.5 µL. Positive controls were spiked with 2 µM of fish REP1.

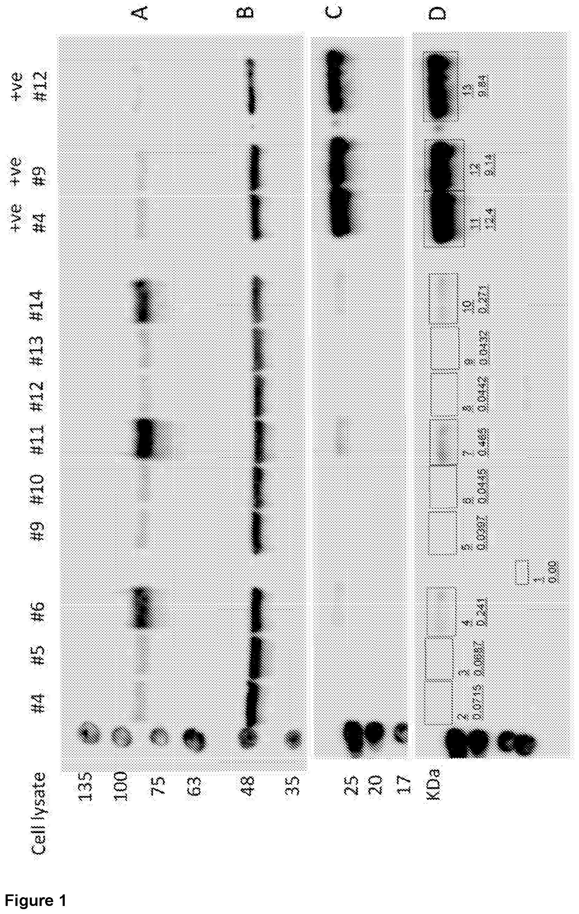

The results indicate that Rab27a is a substrate for the prenylation assay to assess REP1 function following transduction of cells with AAV-REP1 (Figure 1). However, the signal from the WB semiquantification is not very strong.

This study was repeated with an increased amount of total cell protein to increase the WB band intensity.

Prenylation reactions were set up using 30 µg of lysate in a total volume of 22 µL. Positive controls were spiked with 1 µM of fish REP1.

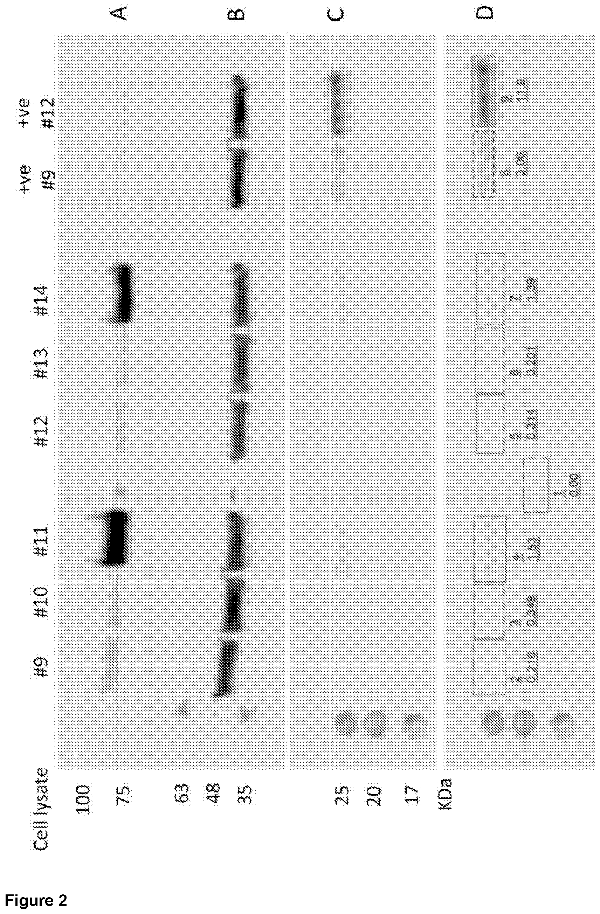

The results confirm that Rab27a works as a substrate for the prenylation assay to assess REP1 function following transduction of cells with AAV-REP1 (Figure 2). Furthermore, the strength of the WB signal has increased compared to the data obtained using 10 µg of lysate. However, the signal is still not very strong. Ideally, a larger increase of prenylated Rab protein when cells are transduced with AAV-REP1 would be observed.

### Example 2 - Rab6a as a prenylation substrate

To test the sensitivity of a prenylation assay using Rab6a as a substrate, experiments were carried out in parallel using the following cells (same cell lysates used in Example 1):
(a) untransduced cells;
(b) cells transduced with AAV-GFP at a MOI of 10000; and
(c) cells transduced with AAV-REP1 at a MOI of 10000.

Prenylation reactions were set up using 20 µg of lysate in a total volume of 20 µL. Positive controls were spiked with 1 µM of fish REP1.

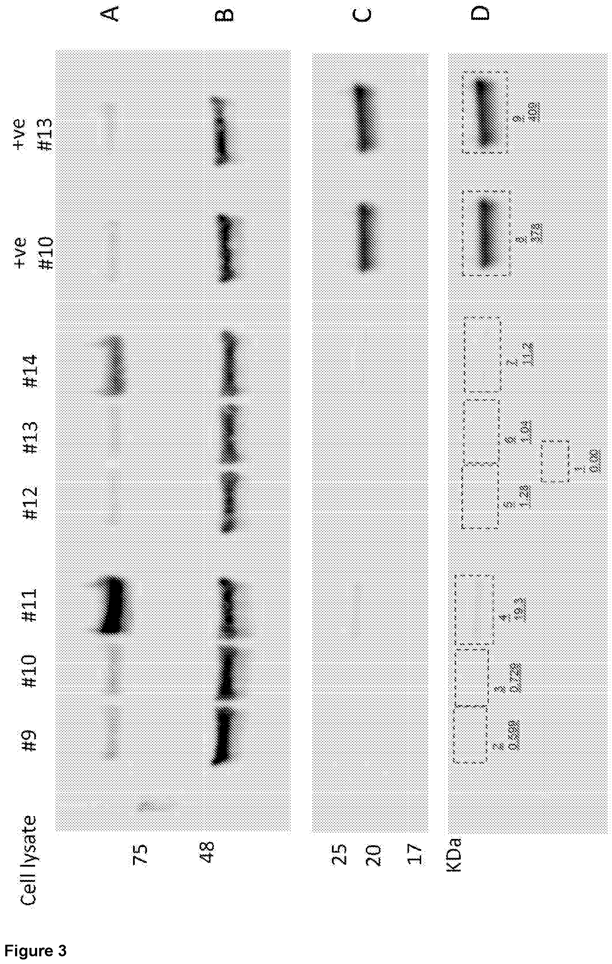

The results indicate that Rab6a is an effective substrate for the prenylation assay to assess REP1 function following transduction of cells with AAV-REP1 (Figure 3).

The strength of the WB signal has increased approximately 10-fold for AAV-REP1 transduced cells, compared to the data shown in Figure 2, even though less total protein was used. Furthermore, the band intensity for the positive controls is approximately 100-fold greater compared to the data shown in Figure 2, confirming the increased sensitivity of the Rab6abased assays.

The data also demonstrate the increased sensitivity enables the detection of differences at endogenous levels too, which makes the assay more accurate.

Following the successful demonstration of increased assay sensitivity provided by the use of Rab6a as the prenylation substrate, we repeated the assay using different MOIs of AAV-REP1 study whether Rab6a prenylation correlates with the amount of AAV-REP1.

Experiments were carried out in parallel using the following cells:
(a) untransduced cells;
(b) cells transduced with AAV-REP1 at a MOI of 250;
(c) cells transduced with AAV-REP1 at a MOI of 1000;
(d) cells transduced with AAV-REP1 at a MOI of 5000;
(e) cells transduced with AAV-REP1 at a MOI of 10000; and
(f) cells transduced with AAV-REP1 at a MOI of 20000.

Prenylation reactions were set up using 20 µg of lysate in a total volume of 15 µL. The positive control was spiked with 0.5 µM of fish REP1.

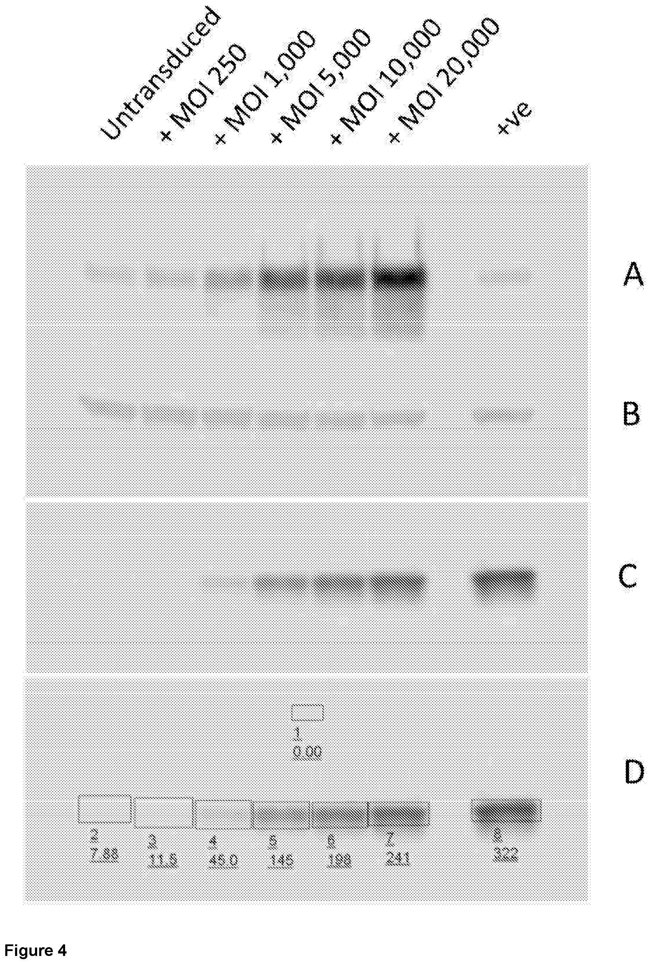

The results confirm that Rab6a is an effective substrate for the prenylation assay to assess REP1 function following transduction of cells with AAV-REP1 (Figure 4) and furthermore demonstrate that the incorporation of biotinylated lipid donor in Rab6a correlates with the amount of AAV-REP1 used for cell transduction.

Following the validation of Rab6a as an effective assay substrate, we then tested the AAV-REP1 vector currently in use in our Phase 1 clinical trial (MacLaren, R.E. et al. (2014) Lancet 383: 1129-37).

Experiments were carried out in parallel using the following cells:
(a) untransduced cells (#29, #30 and #31);
(b) cells transduced with AAV-REP1 at a MOI of 10000 (#32, #33 and #34); and
(c) cells transduced with GMP grade AAV-REP1 at a MOI of 10000 (#35, #36 and #37).

Prenylation reactions were set up using 20 µg of lysate in a total volume of 15 µL. The positive control was spiked with 0.5 µM of fish REP1.

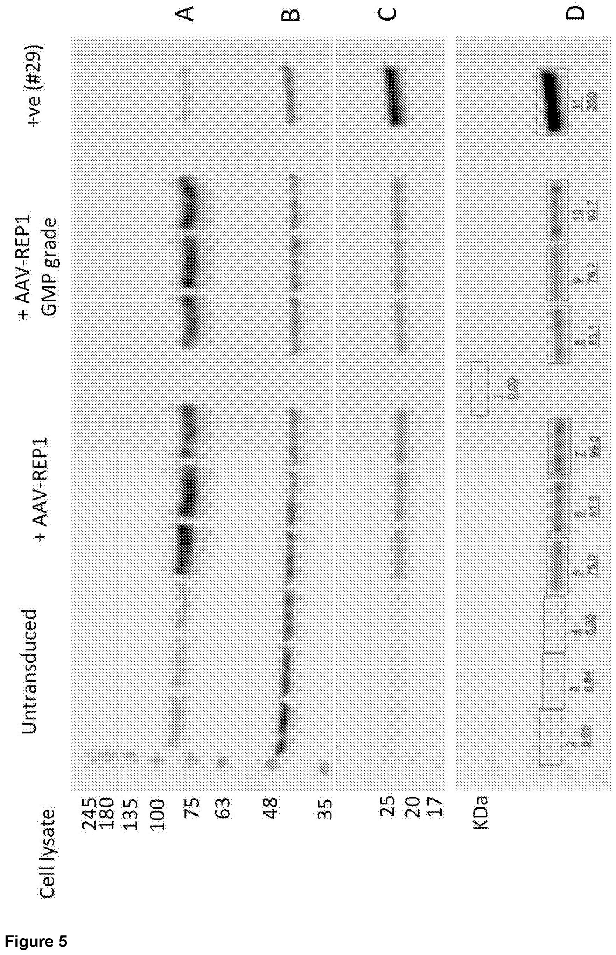

The results are in keeping with the previous experiments and confirm that the incorporation of biotinylated lipid donor in Rab6a correlates with the amount of AAV-REP1 used for cell transduction (Figure 5).

### Example 3 - Rab6a as a substrate in the prenylation reaction using ARPE-19 cells

### Cell transduction and harvesting

Cultured ARPE-19 cells were treated with rAAV2/2-REP1 at an MOI of 10,000 genome particles/cell. Cell lysates were prepared at day 13 post-transduction: cells were washed with PBS and incubated with prenylation buffer, pH 7.5 (50 mM HEPES, 50 mM NaCl, 2 mM MgCl₂, 1 mM DTT and protease inhibitor cocktail (Roche)) on ice. Cells were scraped using a cell scraper into a 1.5 mL tube, and incubated on ice for 15 min. Cells were disrupted by pushing them 20 times through a 26-G syringe needle attached to a 1 mL syringe. Cells were spun for 5 min at 1,500 g, 4°C. The supernatant was then transferred to cellulose propionate tubes and centrifuged at 100,000 g for 1 h at 4°C. The supernatant was used for the in vitro prenylation reaction.

### Total protein quantification

Total cell protein was quantified using the Bradford method according to the manufacturer's instructions (Quick Start™ Bradford 1x Dye Reagent, BioRad, #500-0205).. Sample values were extrapolated from a standard curve.

### In vitro prenylation reaction

The prenylation reactions were set up using frozen cell lysate (15 µg), 2 µM Rab GGTase, 4 µM of Rab protein (Rab6a) and 5 µM of biotin-geranylpyrophosphate as lipid donor, in prenylation buffer. All reactions were supplemented with fresh GDP (20 µM) and DTT (1 mM). In positive control samples, fish REP1 (see experiments for amount) was added to the prenylation reaction containing untransduced cell lysate.

The reactions were incubated for 2 h at 37°C and then stopped by addition of SDS-PAGE sample buffer.

Western blotting (WB) was performed to detect human REP-1, β-actin (loading control) and biotinylated Rab protein (Rab27a or Rab6a). For detection of human REP1, a mouse monoclonal antibody from Millipore was used (clone 2F1, #MABN52). For detection of β-actin, a mouse monoclonal antibody from Thermo Fisher Scientific was used (clone AC-15, # AM4302). Both detections were followed by a secondary antibody-labelling step (donkey anti-mouse HRP, Abcam, #ab98799). The incorporation of biotinylated lipid donor into the appropriate Rab substrate was detected by direct incubation with streptavidin-HRP (Thermo Fisher Scientific, #43-4323). All membranes were detected using ECL substrate and Odyssey FC detection system (LI-COR). The intensities of the bands were quantitatively analysed using Image Studi Lite software (LI-COR).

### Results and discussion

To test the prenylation assay using Rab6a as a substrate in ARPE-19 cells (human retinal pigment epithelium cells), experiments were carried out in parallel using the following cells:
(a) Untransduced cells (#86 and #87); and
(b) Cells + AAV-REP1 MOI 10,000 (#90 and #91) - R&D grade vector.

Prenylation reactions were set up using 15 µg of lysate in a total volume of 45 µL. Positive control was spiked with 0.1 µM of fish REP1.

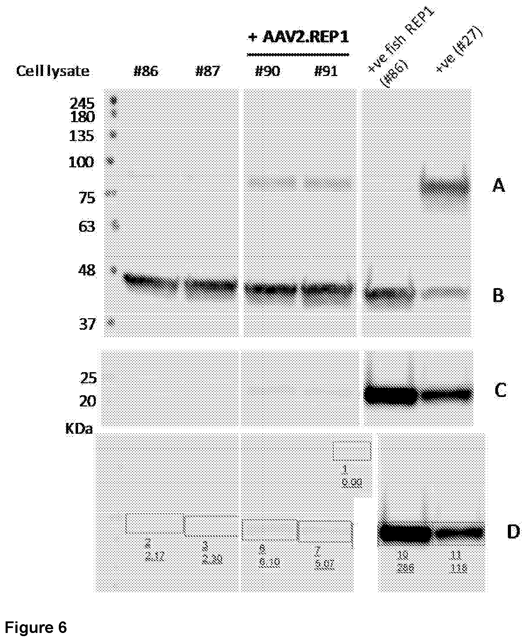

The results indicate that Rab6a works as a substrate for the prenylation assay to assess REP1 function following transduction of ARPE-19 cells with AAV-REP1 (Figure 6).

### Example 4 - Rab6a as a substrate in the prenylation reaction using HT1080 cells

### Cell transduction and harvesting

Cultured HT1080 cells were treated with rAAV2/2-REP1 at an MOI of 10,000 genome particles/cell. Cell lysates were prepared at day 5 post-transduction: cells were washed with PBS and incubated with prenylation buffer, pH 7.5 (50 mM HEPES, 50 mM NaCl, 2 mM MgCl₂, 1 mM DTT and protease inhibitor cocktail (Roche)) on ice. Cells were scraped using a cell scraper into a 1.5 mL tube, and incubated on ice for 15 min. Cells were disrupted by pushing them 20 times through a 26-G syringe needle attached to a 1 mL syringe. Cells were spun for 5 min at 1,500 g, 4°C. The supernatant was then transferred to cellulose propionate tubes and centrifuged at 100,000 g for 1 h at 4°C. The supernatant was used for the in vitro prenylation reaction.

### Total protein quantification

Total cell protein was quantified using the Bradford method according to the manufacturer's instructions (Quick Start™ Bradford 1x Dye Reagent, BioRad, #500-0205). Sample values were extrapolated from a standard curve.

### In vitro prenylation reaction

The prenylation reactions were set up using frozen cell lysate (20 µg), 2 µM Rab GGTase, 4 µM of Rab protein (Rab6a) and 5 µM of biotin-geranylpyrophosphate as lipid donor, in prenylation buffer. All reactions were supplemented with fresh GDP (20 µM) and DTT (1 mM). In positive control samples, fish REP1 (see experiments for amount) was added to the prenylation reaction containing untransduced cell lysate.

The reactions were incubated for 2 h at 37°C and then stopped by addition of SDS-PAGE sample buffer.

Western blotting (WB) was performed to detect human REP-1, β-actin (loading control) and biotinylated Rab protein (Rab27a or Rab6a). For detection of human REP1, a mouse monoclonal antibody from Millipore was used (clone 2F1, #MABN52). For detection of β-actin, a mouse monoclonal antibody from Thermo Fisher Scientific was used (clone AC-15, # AM4302). Both detections were followed by a secondary antibody-labelling step (donkey anti-mouse HRP, Abcam, #ab98799). The incorporation of biotinylated lipid donor into the appropriate Rab substrate was detected by direct incubation with streptavidin-HRP (Thermo Fisher Scientific, #43-4323). All membranes were detected using ECL substrate and Odyssey FC detection system (LI-COR). The intensities of the bands were quantitatively analysed using Image Studi Lite software (LI-COR).

### Results and discussion

To test the prenylation assay using Rab6a as a substrate in HT1080 cells, experiments were carried out in parallel using the following cells:
(a) Untransduced cells (#56 and #57);
(b) Cells + AAV-REP1 MOI 10,000 (#60 and #61) - R&D grade vector; and
(c) Cells + AAV-REP1 MOI 10,000 (#64 and #65) - clinical grade vector.

Prenylation reactions were set up using 20 µg of lysate in a total volume of 20 µL. Positive control was spiked with 0.1 µM of fish REP1.

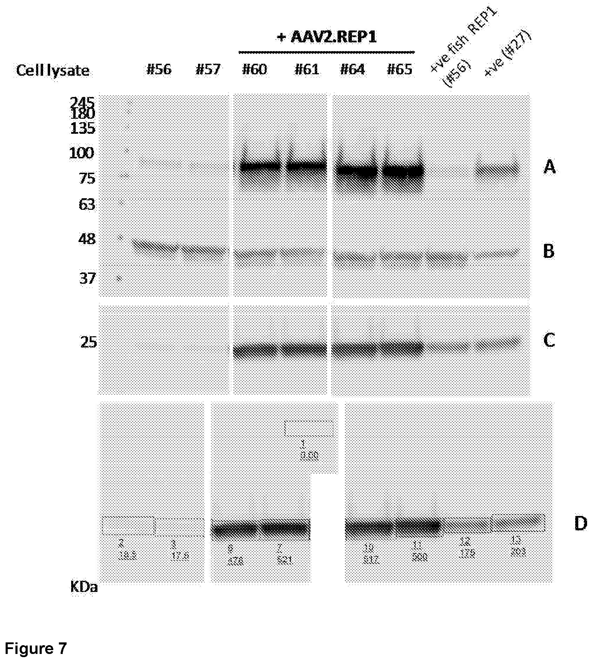

The results indicate that Rab6a works as a substrate for the prenylation assay to assess REP1 function following transduction of HT1080 cells with AAV-REP1 (Figure 7).

### Example 5 - Comparison of Rab27a and Rab6a as substrates in prenylation reactions

The same cell lysates were used as in the experiment shown in Figure 4:
(a) Untransduced cells;
(b) Cells + AAV-REP1 MOI 250;
(c) Cells + AAV-REP1 MOI 1,000;
(d) Cells + AAV-REP1 MOI 5,000;
(e) Cells + AAV-REP1 MOI 10,000; and
(f) Cells + AAV-REP1 MOI 20,000.

Prenylation reactions were set up using 20 µg of lysate in a total volume of 15 µL, and 2 different substrates: Rab27a and Rab6a. Positive controls, one for each substrate, were spiked with 0.1 µM of fish REP1. Samples were run in parallel on SDS-PAGE and detected simultaneously.

### Results and discussion

Both Rab27a and Rab6a work as a substrate for the prenylation assay to assess REP1 function following transduction of cells with AAV-REP1.

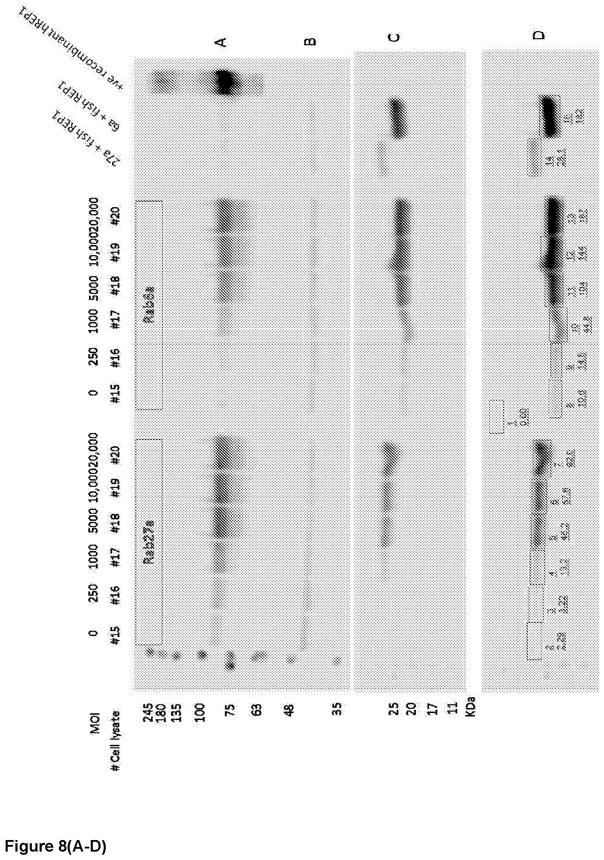

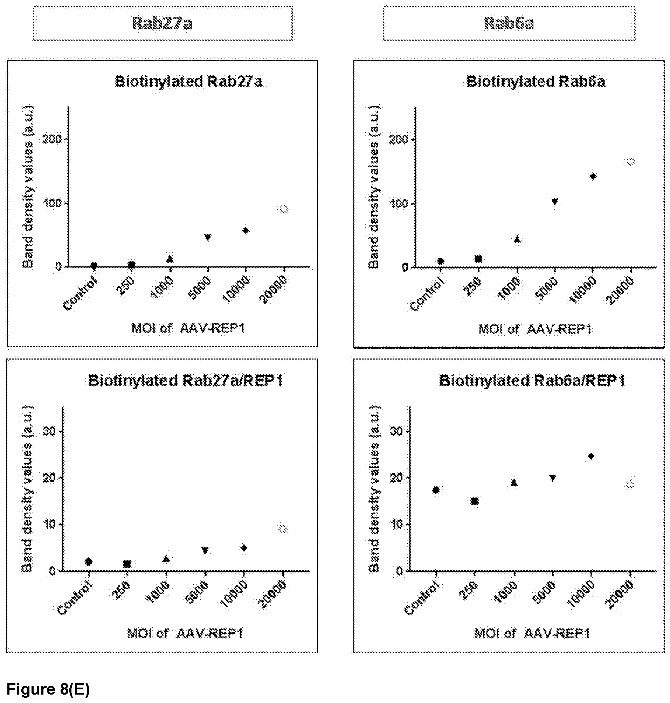

The incorporation of biotinylated lipid donor correlates with the amount of AAV-REP1 used for cell transduction for each of the substrates used (Figure 8).

The band density from biotinylated Rab6a is higher than for Rab27a, which indicates Rab6a is a more suitable substrate for a parallel line analysis for determination of relative potency.

### Example 6 - Comparison of Rab27a and Rab6a performance as substrates in prenylation reactions using different conditions

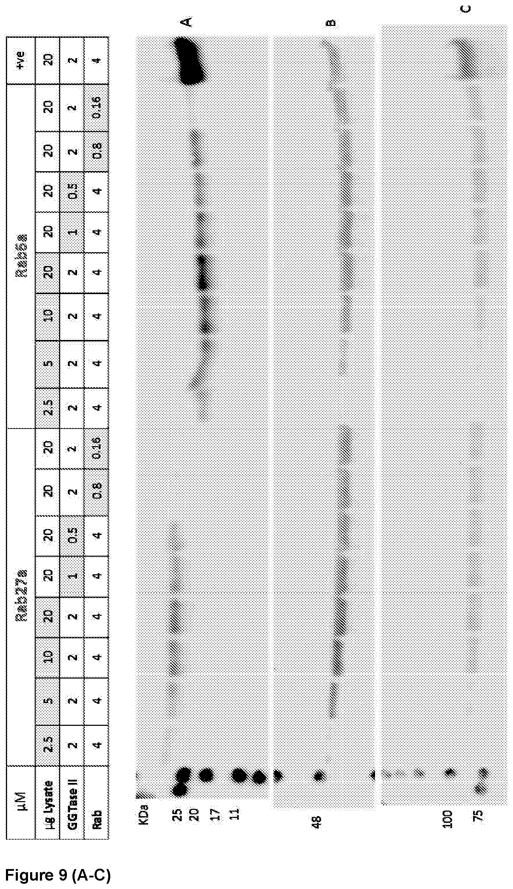

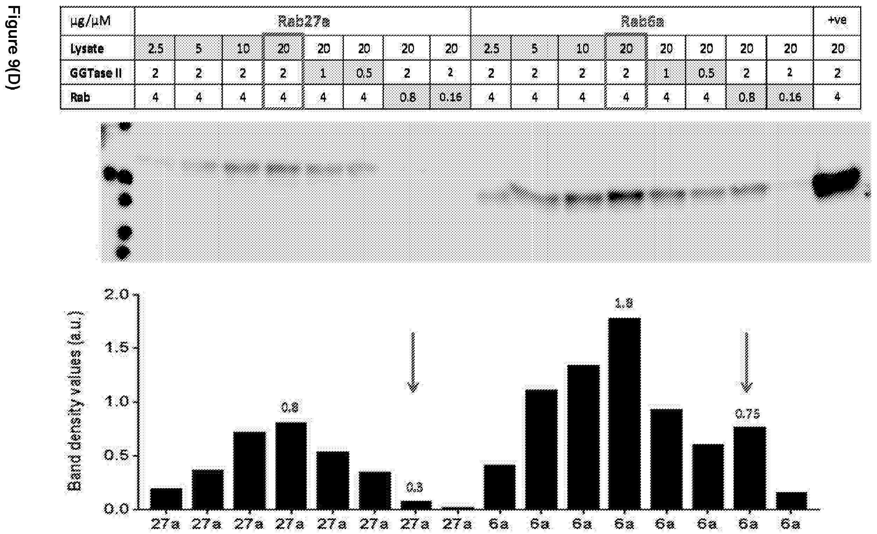

Untransduced lysate of 293 cells was prepared for use in this experiment using Rab27a and Rab6a. The conditions tested are shown in the tables in Figure 9. Samples were run in parallel on SDS-PAGE and detected simultaneously.

### Results and discussion

Both Rab27a and Rab6a work as a substrate for the prenylation assay to assess endogenous REP1 function.

The incorporation of biotinylated lipid donor correlates with the amount of total protein in the reaction for each of the substrates used.

Comparing the conditions, the concentration of Rab substrate in the reaction seems to affect the signal the most.

There is a 2.5-fold increase in the biotinylated substrate when Rab6a is used, compared to Rab27a.

### Example 7 - Comparison of Rab27a and Rab6a as substrates in prenylation reactions in lysates transduced with AAV2-REP1

New lysates (in triplicate) were prepared using increasing MOIs of AAV2-REP1 (R&D material):
(a) Untransduced cells;
(b) Cells + AAV-REP1 MOI 100;
(c) Cells + AAV-REP1 MOI 500;
(d) Cells + AAV-REP1 MOI 1,000;
(e) Cells + AAV-REP1 MOI 5,000;
(f) Cells + AAV-REP1 MOI 10,000;
(g) Cells + AAV-REP1 MOI 20,000; and
(h) Cells + AAV-REP1 MOI 50,000.

Prenylation reactions were prepared using 20 µg of total protein, 2 µM of Rab substrate (Rab27a or Rab6a) and 2 µM of Rab GGTase, in a total volume of 10 µL. Positive controls, one for each substrate, were spiked with 0.1 µM of fish REP1.

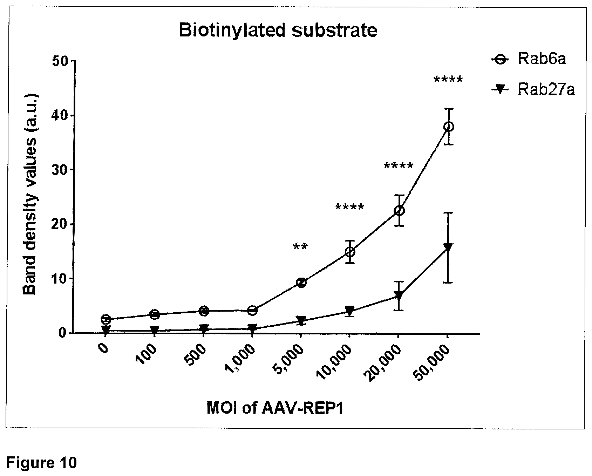

Samples from each replicate were run in parallel on SDS-PAGE and detected for biotinylated substrate (1:10,000), β-actin (1:50,000) as loading control and human REP1 (1:2,500) using Image Studio Lite software. Data from semiquantification of band density for biotinylated substrate was plotted using Prism software (Figure 10).

### Results

The levels of β-actin were similar in all samples analysed. Untransduced cells (and positive control samples) showed endogenous level of REP1. Cells transduced with AAV-REP1 showed an increase of REP1 levels that directly correlates with the MOI used. Positive controls show stronger biotin incorporation, as a result of fish REP1 activity.

A two-way ANOVA analysis of all three replicates with substrate and MOI as factors found that both were highly significant (*p*<0.0001). Bonferroni's multiple comparisons test for the effect of the substrate at a given MOI showed a significant pairwise difference at MOI of 5,000 (p=0.0023) and all above (*p*<0.0001).

### Discussion

Both Rab27a and Rab6a work as a substrate for the prenylation assay to assess REP1 function following transduction of cells with AAV-REP1.

Semiquantification of band density for biotinylated substrate only shows the values for Rab6a to be significantly higher than those obtained for Rab27a.

## Claims

1. A method for determining the activity of a Rab escort protein 1 (REP1)-encoding gene therapy vector for the use in the treatment of choroideremia, the method comprising the steps of:
(a) contacting a sample comprising REP1 with Rab6a, Rab geranylgeranyltransferase (Rab GGTase) and a lipid donor substrate, wherein the sample comprising REP1 is from a cell genetically engineered to express the REP1 using the REP1-encoding gene therapy vector; and
(b) detecting the lipidated Rab6a product.

2. A method for quality control analysis of a Rab escort protein 1 (REP1)- encoding gene therapy vector for the use in the treatment of choroideremia, the method comprising the steps of:
(a) contacting a sample comprising REP1 with Rab6a, Rab geranylgeranyltransferase (Rab GGTase) and a lipid donor substrate, wherein the sample comprising REP1 is from a cell genetically engineered to express the REP1 using the REP1-encoding gene therapy vector; and
(b) detecting the lipidated Rab6a product.

3. The method of claim 1 or 2, wherein the sample comprising REP1 is a lysate of the cell genetically engineered to express the REP1 using the REP1-encoding gene therapy vector.

4. The method of any one of claims 1-3, wherein the REP1-encoding gene therapy vector is a viral vector comprising a REP1-encoding nucleotide sequence, preferably wherein the viral vector is an adeno-associated viral (AAV) vector, preferably wherein the viral vector is AAV serotype 2 (AAV2).

5. The method of any preceding claim, wherein the REP-1 encoding nucleotide sequence of the REP1-encoding gene therapy vector comprises a nucleotide sequence that encodes an amino acid sequence that has at least 80% identity to SEQ ID NO: 5, preferably wherein the amino acid sequence substantially retains the natural function of the protein represented by SEQ ID NO: 5.

6. The method of any preceding claim, wherein the Rab6a and/or Rab GGTase are substantially pure.

7. The method of any preceding claim, wherein the Rab6a:Rab GGTase molar ratio is 1:2.3 or 1:2.5.

8. The method of any preceding claim, wherein the lipid donor substrate is geranylgeranylpyrophosphate (GGPP) or an analogue thereof, preferably biotin-geranylpyrophosphate (BGPP).

9. The method of any preceding claim, wherein the lipidated Rab6a product is detected using an enzyme-linked immunosorbent assay (ELISA), Western blot analysis or autoradiography.

10. The method of any preceding claim, wherein the detection of the lipidated Rab6a product comprises quantifying the amount of the lipidated Rab6a product, preferably quantifying the amount relative to a control or reference level.

11. Use of Rab6a for determining in vitro the activity of a Rab escort protein 1 (REP1)-encoding gene therapy vector for use in the treatment of choroideremia, wherein the Rab6a is contacted with REP1 from a cell genetically engineered to express the REP1 using the REP1-encoding gene therapy vector.

12. Use of Rab6a for in vitro quality control analysis of a Rab escort protein 1 (REP1)-encoding gene therapy vector for the use in the treatment of choroideremia, wherein the Rab6a is contacted with REP1 from a cell genetically engineered to express the REP1 using the REP1-encoding gene therapy vector.

13. The use of claim 11 or 12, wherein the REP1 is in a lysate from the cell genetically engineered to express the REP1 using the REP1-encoding gene therapy vector.

14. The use of any one of claims 11-13, wherein the REP1-encoding gene therapy vector is a viral vector comprising a REP1-encoding nucleotide sequence, preferably wherein the viral vector is an adeno-associated viral (AAV) vector.

15. The use of any one of claims 11-14, wherein the Rab6a is substantially pure.

## Patentansprüche

1. Verfahren zur Bestimmung der Aktivität eines ein Rab-Escort-Protein 1 (REP1) codierenden Gentherapievektors zur Verwendung bei der Behandlung von Choroiderämie, wobei das Verfahren die folgenden Schritte umfasst:
(a) das In-Kontakt-Bringen einer REP1 umfassenden Probe mit Rab6a, Rab-Geranylgeranyltransferase (Rab GGTase) und einem Lipiddonorsubstrat, wobei die REP1 umfassende Probe von einer Zelle stammt, die so gentechnisch verändert ist, dass sie das REP1 unter Verwendung des REP1 codierenden Gentherapievektors exprimiert; und
(b) das Detektieren des lipidierten Rab6a-Produkts.

2. Verfahren zur Qualitätskontrollanalyse eines ein Rab-Escort-Protein 1 (REP1) codierenden Gentherapievektors zur Verwendung bei der Behandlung von Choroiderämie, wobei das Verfahren die folgenden Schritte umfasst:
(a) das In-Kontakt-Bringen einer REP1 umfassenden Probe mit Rab6a, Rab-Geranylgeranyltransferase (Rab-GGTase) und einem Lipiddonorsubstrat, wobei die REP1 umfassende Probe von einer Zelle stammt, die so gentechnisch verändert ist, dass sie das REP1 unter Verwendung des REP1 codierenden Gentherapievektors exprimiert; und
(b) das Detektieren des lipidierten Rab6a-Produkts.

3. Verfahren nach Anspruch 1 oder 2, wobei die REP1 umfassende Probe ein Lysat der Zelle ist, die so gentechnisch verändert ist, dass sie das REP1 unter Verwendung des REP1-codierenden Gentherapievektors exprimiert.

4. Verfahren nach einem der Ansprüche 1-3, wobei der REP1 codierende Gentherapievektor ein viraler Vektor ist, der eine REP1 codierende Nucleotidsequenz umfasst, wobei der virale Vektor vorzugsweise ein adenoassoziierter viraler (AAV-)Vektor ist, wobei der virale Vektor vorzugsweise der AAV-Serotyp 2 (AAV2) ist.

5. Verfahren nach einem der vorhergehenden Ansprüche, wobei die REP1 codierende Nucleotidsequenz des REP1 codierenden Gentherapievektors eine Nucleotidsequenz umfasst, die eine Aminosäuresequenz codiert, deren Identität mit SEQ ID Nr.: 5 mindestens 80 % beträgt, wobei die Aminosäuresequenz vorzugsweise im Wesentlichen die natürliche Funktion des durch SEQ ID Nr.: 5 dargestellten Proteins beibehält.

6. Verfahren nach einem der vorhergehenden Ansprüche, wobei das Rab6a und/oder die Rab-GGTase im Wesentlichen rein sind.

7. Verfahren nach einem der vorhergehenden Ansprüche, wobei das Stoffmengenverhältnis Rab6a:Rab-GGTase 1:2,3 oder 1:2,5 beträgt.

8. Verfahren nach einem der vorhergehenden Ansprüche, wobei das Lipiddonorsubstrat Geranylgeranylpyrophosphat (GGPP) oder ein Analog davon, vorzugsweise Biotin-Geranylpyrophosphat (BGPP), ist.

9. Verfahren nach einem der vorhergehenden Ansprüche, wobei das lipidierte Rab6a-Produkt unter Verwendung eines enzymgekoppelten Immunadsorptionsassays (ELISA), einer Western-Blot-Analyse oder einer Autoradiographie detektiert wird.

10. Verfahren nach einem der vorhergehenden Ansprüche, wobei die Detektion des lipidierten Rab6a-Produkts das Quantifizieren der Menge des lipidierten Rab6a-Produkts, vorzugsweise das Quantifizieren der Menge in Bezug auf einen Kontroll- oder Bezugsspiegel, umfasst.

11. Verwendung von Rab6a zur In-vitro-Bestimmung der Aktivität eines Rab-Escort-Protein 1 (REP1) codierenden Gentherapievektors zur Verwendung bei der Behandlung von Choroiderämie, wobei das Rab6a mit REP1 aus einer Zelle in Kontakt gebracht wird, die so gentechnisch verändert ist, dass sie das REP1 unter Verwendung des REP1 codierenden Gentherapievektors exprimiert.

12. Verwendung von Rab6a zur In-vitro-Qualitätskontrollanalyse eines Rab-Escort-Protein 1 (REP1) codierenden Gentherapievektors zur Verwendung bei der Behandlung von Choroiderämie, wobei das Rab6a mit REP1 aus einer Zelle in Kontakt gebracht wird, die so gentechnisch verändert ist, dass sie das REP1 unter Verwendung des REP1 codierenden Gentherapievektors exprimiert.

13. Verwendung nach Anspruch 11 oder 12, wobei das REP1 in einem Lysat der Zelle vorliegt, die so gentechnisch verändert ist, dass sie das REP1 unter Verwendung des REP1-codierenden Gentherapievektors exprimiert.

14. Verwendung nach einem der Ansprüche 11-13, wobei der REP1 codierende Gentherapievektor ein viraler Vektor ist, der eine REP1 codierende Nucleotidsequenz umfasst, wobei der virale Vektor vorzugsweise ein adeno-assoziierter viraler (AAV-)Vektor ist.

15. Verwendung nach einem der Ansprüche 11-14, wobei das Rab6a im Wesentlichen rein ist.

## Revendications

1. Procédé pour déterminer l'activité d'un vecteur de thérapie génique codant pour la protéine d'escorte Rab 1 (REP1), pour une utilisation dans le traitement de la choroïdérémie, le procédé comprenant les étapes de :
(a) mise en contact d'un échantillon comprenant la REP1 avec la Rab6a, la Rab géranylgéranyltransférase (Rab GGTase) et un substrat donneur de lipides, l'échantillon comprenant la REP1 provenant d'une cellule génétiquement modifiée pour exprimer la REP1 par utilisation du vecteur de thérapie génique codant pour la REP1 ; et
(b) détection du produit Rab6a lipidé.

2. Procédé pour l'analyse de contrôle de qualité d'un vecteur de thérapie génique codant pour la protéine d'escorte Rab 1 (REP1) pour une utilisation dans le traitement de la choroïdérémie, le procédé comprenant les étapes de :
(a) mise en contact d'un échantillon comprenant la REP1 avec la Rab6a, la Rab géranylgéranyltransférase (Rab GGTase) et un substrat donneur de lipides, l'échantillon comprenant la REP1 provenant d'une cellule génétiquement modifiée pour exprimer la REP1 par utilisation du vecteur de thérapie génique codant pour la REP1 ; et
(b) détection du produit Rab6a lipidé.

3. Procédé selon la revendication 1 ou 2, dans lequel l'échantillon comprenant la REP1 est un lysat de la cellule génétiquement modifiée pour exprimer la REP1 par utilisation du vecteur de thérapie génique codant pour la REP1.

4. Procédé selon l'une quelconque des revendications 1 à 3, dans lequel le vecteur de thérapie génique codant pour la REP1 est un vecteur viral comprenant une séquence nucléotidique codant pour la REP1, de préférence dans lequel le vecteur viral est un vecteur viral adéno-associé (AAV), de préférence dans lequel le vecteur viral est un AAV sérotype 2 (AAV2).

5. Procédé selon l'une quelconque des revendications précédentes, dans lequel la séquence nucléotidique codant pour la REP1 du vecteur de thérapie génique codant pour la REP1 comprend une séquence nucléotidique qui code pour une séquence d'acides aminés qui a une identité d'au moins 80 % avec la SEQ ID NO: 5, de préférence dans lequel la séquence d'acides aminés conserve pratiquement la fonction naturelle de la protéine représentée par SEQ ID NO: 5.

6. Procédé selon l'une quelconque des revendications précédentes, dans lequel la Rab6a et/ou la Rab GGTase sont pratiquement pures.

7. Procédé selon l'une quelconque des revendications précédentes, dans lequel le rapport en moles Rab6a:Rab GGTase est de 1:2,3 ou de 1:2,5.

8. Procédé selon l'une quelconque des revendications précédentes, dans lequel le substrat donneur de lipides est le pyrophosphate de géranylgéranyle (GGPP) ou un analogue de celui-ci, de préférence le pyrophosphate de biotine-géranyle (BGPP).

9. Procédé selon l'une quelconque des revendications précédentes, dans lequel le produit Rab6a lipidé est détecté par utilisation d'une analyse par immunosorbant à liaison enzymatique (ELISA), d'une analyse par transfert de Western ou d'une autoradiographie.

10. Procédé selon l'une quelconque des revendications précédentes, dans lequel la détection du produit Rab6a lipidé comprend la quantification de la quantité du produit Rab6a lipidé, de préférence la quantification de la quantité par rapport à un niveau témoin ou de référence.

11. Utilisation de la Rab6a pour déterminer in vitro l'activité du vecteur de thérapie génique codant pour la protéine d'escorte Rab 1 (REP1) pour une utilisation dans le traitement de la choroidérémie, dans laquelle la Rab6a est mise en contact avec la REP1 provenant d'une cellule génétiquement modifiée pour exprimer la REP1 par utilisation du vecteur de thérapie génique codant pour la REP1.

12. Utilisation de la Rab6a pour une analyse de contrôle de qualité in vitro d'un vecteur de thérapie génique codant pour la protéine d'escorte Rab 1 (REP1) pour une utilisation dans le traitement de la choroïdérémie, dans laquelle la Rab6a est mise en contact avec la REP1 provenant d'une cellule génétiquement modifiée pour exprimer la REP1 par utilisation du vecteur de thérapie génique codant pour la REP1.

13. Utilisation selon la revendication 11 ou 12, dans laquelle la REP1 est un lysat provenant de la cellule génétiquement modifiée pour exprimer la REP1 par utilisation du vecteur de thérapie génique codant pour la REP1.

14. Utilisation selon l'une quelconque des revendications 11 à 13, dans laquelle le vecteur de thérapie génique codant pour la REP1 est un vecteur viral comprenant une séquence nucléotidique codant pour la REP1, de préférence dans laquelle le vecteur viral est un vecteur viral adéno-associé (AAV).

15. Utilisation selon l'une quelconque des revendications 11 à 14, dans laquelle la Rab6a est pratiquement pure.
